# EUROPEAN PATENT APPLICATION

(11) **EP 4 610 997 A1**
(43) Date of publication of application: **03.09.2025**
(21) Application number: 24182408.5
(22) Date of filing: 14.06.2024
(51) Int. Cl.: G16H 30/40, G16H 50/20

(54) **BLOOD FLOW FIELD INFORMATION DETERMINATION METHOD, DEVICE, COMPUTING DEVICE, AND STORAGE MEDIUM**

(30) Priority: 01.03.2024 CN 202410239473; 01.03.2024 CN 202410239335; 01.03.2024 CN 202410238842; 01.03.2024 CN 202410239087; 01.03.2024 CN 202410239171
(71) Applicant: Yukun (Beijing) Technology Co., Ltd., Beijing 102209 (CN)
(72) Inventor: XIAO, Yueting, Beijing, 102209 (CN); GAO, Xin, Beijing, 102209 (CN); YANG, Guang, Beijing, 102209 (CN); ZHENG, Chao, Beijing, 102209 (CN); MAO, Xinsheng, Beijing, 102209 (CN); MA, Chune, Beijing, 102209 (CN)
(74) Representative: Lucke, Andreas

(57) **Abstract**

A method, device, computing equipment, and storage medium for determining blood flow field information are provided. The method can include: obtaining vascular segment images regarding a target vascular segment; and based on the vascular segment images, determining at least one blood flow field information for the said target vascular segment through a pre-trained neural network.

## Description

### Technical Field

This disclosure relates to the field of data processing, especially a method, device, computing device, and storage medium for determining blood flow field information.

### Background

In the medical field, various flow field information related to blood can reflect many valuable insights. A method for analyzing or determining blood flow field information is desirable.

The methods described in this section are not necessarily previously conceived or adopted methods. Unless otherwise specified, any method described in this section should not be considered prior art simply because it is included in this section. Similarly, unless otherwise specified, the problems mentioned in this section should not be assumed to have been recognized in any prior art.

### SUMMARY

According to one aspect of this disclosure, a method for determining blood flow field information is provided, including: obtaining vascular segment images regarding a target vascular segment; and determining at least one blood flow field information for the target vascular segment based on the vascular segment images, through a pre-trained neural network. Predicted values of a first physical quantity and a second physical quantity in the blood flow field corresponding to the target vascular segment are obtained based on the vascular segment image data, where the predicted values of the first and second physical quantities satisfy at least one physical constraint condition; and at least one human medical information regarding the target vascular segment is determined based on the predicted value of the first physical quantity. According to another aspect of this disclosure, a device for determining blood flow field information is provided, including: an image acquisition unit for obtaining vascular segment images regarding a target vascular segment; and an information determination unit for determining at least one blood flow field information for the target vascular segment based on the vascular segment images, through a pre-trained neural network.

According to another aspect of this disclosure, a computing device is provided, including: a memory, a processor, and a computer program stored on the memory, where the processor is configured to execute the computer program to implement the blood flow field information determination method according to one or more embodiments of this disclosure.

According to another aspect of this disclosure, a non-transitory computer-readable storage medium is provided, storing a computer program, where the computer program, when executed by a processor, implements the blood flow field information determination method according to one or more embodiments of this disclosure.

According to another aspect of this disclosure, a method for training a blood flow prediction model is provided, including: obtaining vascular segment image data about sample vascular segments; obtaining predicted values of a first physical quantity and a second physical quantity in the blood flow field corresponding to the sample vascular segments, through the blood flow field prediction model, where the first physical quantity can be used to determine at least one human medical information regarding the sample vascular segments; and adjusting the parameters of the blood flow field prediction model based on at least one of the first strategy, the second strategy, and the third strategy, where the first strategy is used to reduce the difference between the predicted value of the first physical quantity and its true value, the second strategy is used to reduce the difference between the predicted value of the second physical quantity and its true value, and the third strategy is used to ensure that the predicted values of the first and second physical quantities satisfy at least one physical constraint condition.

According to another aspect of this disclosure, a human medical information determination device is provided, including: an image acquisition unit for obtaining vascular segment image data regarding a target vascular segment; a physical quantity prediction unit for obtaining predicted values of a first physical quantity and a second physical quantity in the blood flow field corresponding to the target vascular segment, where the predicted values of the first and second physical quantities satisfy at least one physical constraint condition; and an information determination unit for determining at least one human medical information regarding the target vascular segment based on the predicted value of the first physical quantity.

According to another aspect of this disclosure, a training method for a blood flow prediction model is provided, including: an image acquisition unit for obtaining vascular segment image data about sample vascular segments; a physical quantity prediction unit for obtaining predicted values of a first physical quantity and a second physical quantity in the blood flow field corresponding to the sample vascular segments, through the blood flow field prediction model, where the first physical quantity can be used to determine at least one human medical information regarding the sample vascular segments; and a model adjustment unit for adjusting the parameters of the blood flow field prediction model based on at least one of the first strategy, the second strategy, and the third strategy, where the first strategy is used to reduce the difference between the predicted value of the first physical quantity and its true value, the second strategy is used to reduce the difference between the predicted value of the second physical quantity and its true value, and the third strategy is used to ensure that the predicted values of the first and second physical quantities satisfy at least one physical constraint condition.

According to another aspect of this disclosure, a computing device is provided, including: a memory, a processor, and a computer program stored on the memory, where the processor is configured to execute the computer program to implement at least one of the human medical information determination method and the blood flow prediction model training method according to one or more embodiments of this disclosure.

According to another aspect of this disclosure, a non-transitory computer-readable storage medium is provided, storing a computer program, where the computer program, when executed by a processor, implements at least one of the human medical information determination method and the blood flow prediction model training method according to one or more embodiments of this disclosure.

According to another aspect of this disclosure, a computer program product is provided, including a computer program, where the computer program, when executed by a processor, implements the blood flow field information determination method according to one or more embodiments of this disclosure.

These and other aspects of this disclosure will be clear and elucidated upon reference to the embodiments described below.

### BRIEF DESCRIPTION OF THE DRAWINGS

In the description of exemplary embodiments that follows, with reference to the accompanying drawings, further details, features, and advantages of this disclosure are disclosed, wherein:
Figure 1 illustrates a schematic of an example system in which various methods described herein can be implemented according to an exemplary embodiment;
Figure 2 is a flowchart illustrating a method for determining blood flow field information according to an exemplary embodiment;
Figure 3A, Figure 3B and Figure 3C illustrate schematics of data flow according to exemplary embodiments;
Figure 3D and Figure 3E illustrate schematics of exemplary vascular segments;

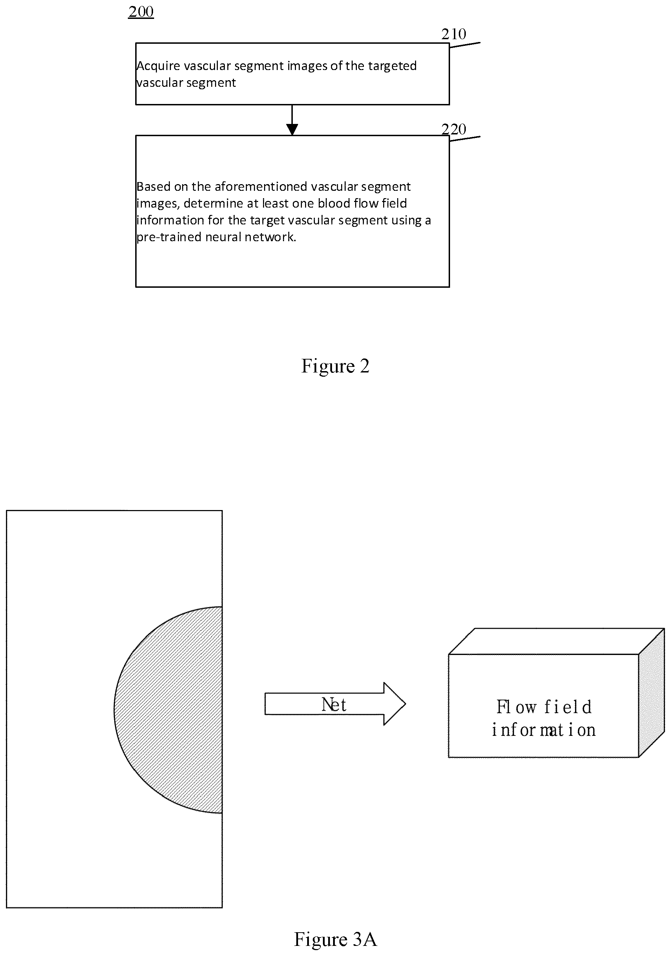

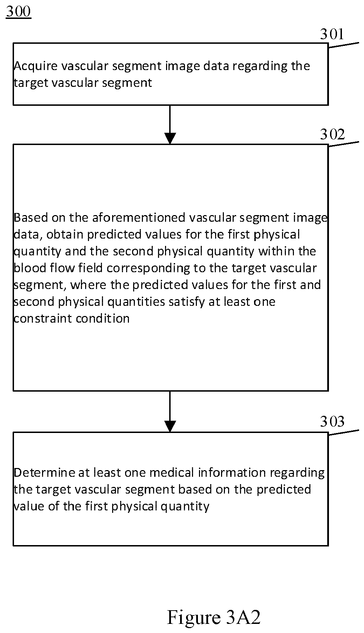

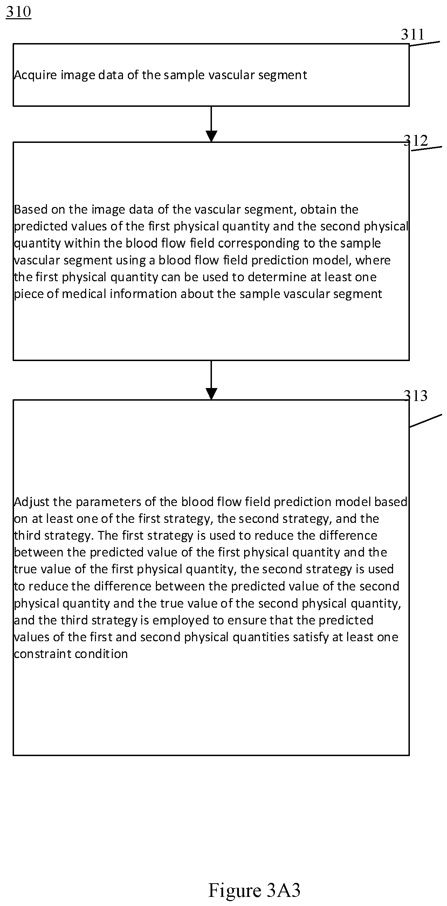

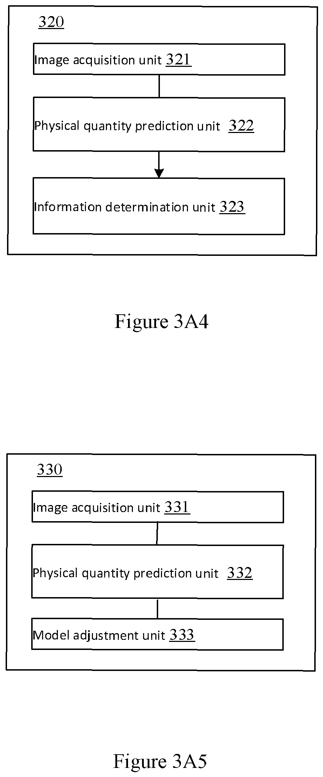

Figure 3A2 is a flowchart illustrating a method for determining human medical information according to an exemplary embodiment;
Figure 3A3 is a flowchart illustrating a method for training a blood flow prediction model according to an exemplary embodiment;
Figure 3A4 is a schematic diagram of a device for determining human medical information according to an exemplary embodiment;
Figure 3A5 is a schematic diagram of a device for training a blood flow prediction model according to an exemplary embodiment; and

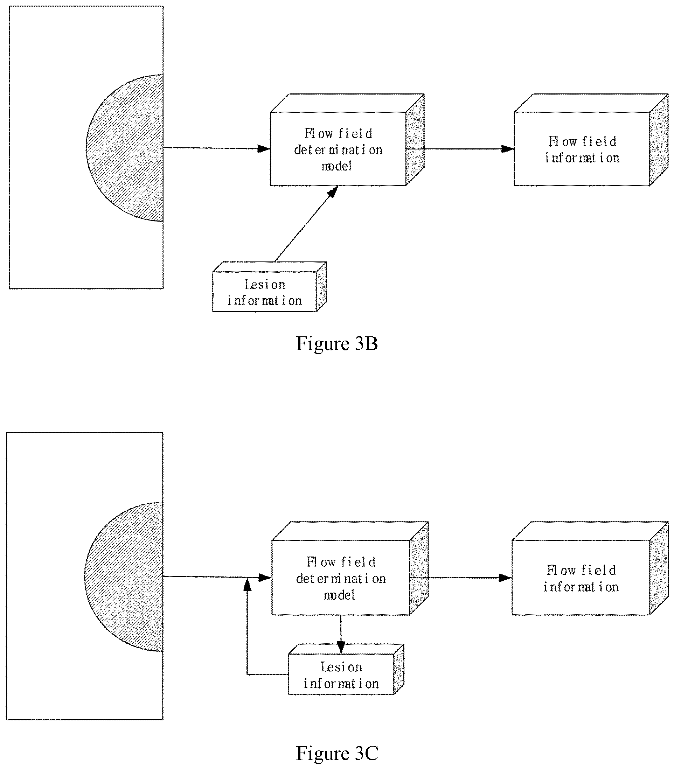

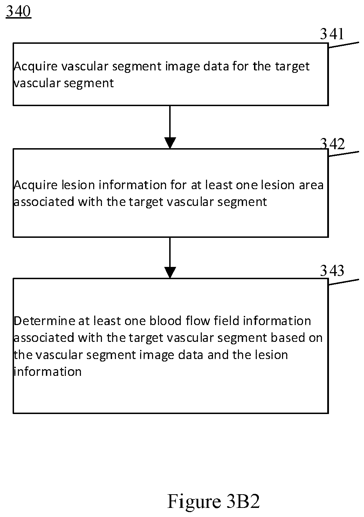

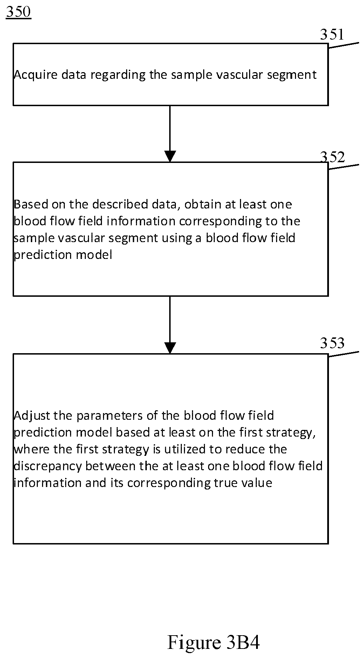

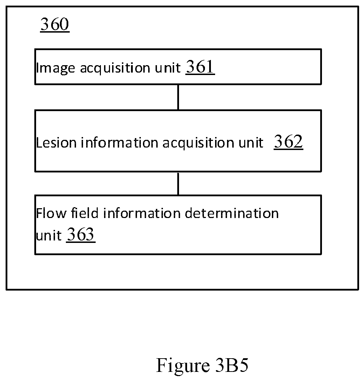

Figure 3B2 is a flowchart illustrating a method for determining blood flow field information based on an exemplary embodiment.
Figure 3B4 is a flowchart illustrating a training method for a blood flow field prediction model based on an exemplary embodiment.
Figure 3B5 is a schematic block diagram of a blood flow field information determination device based on an exemplary embodiment.

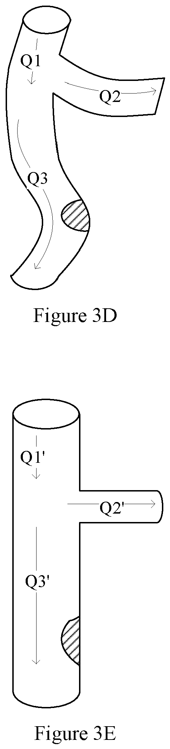

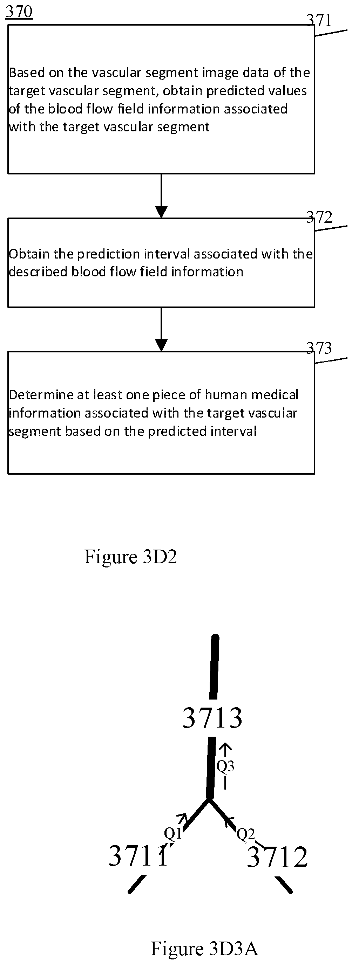

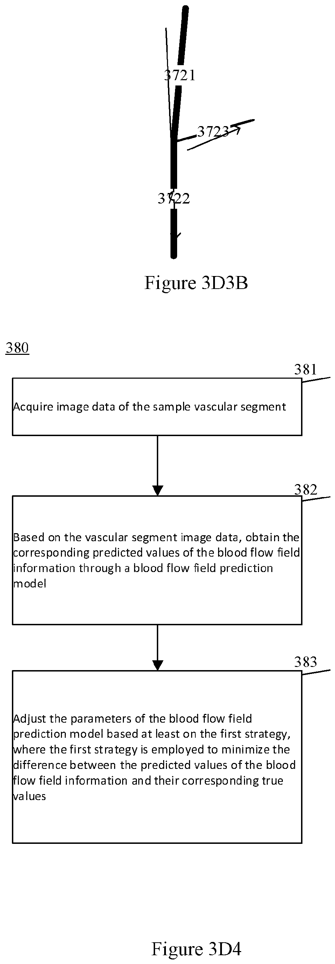

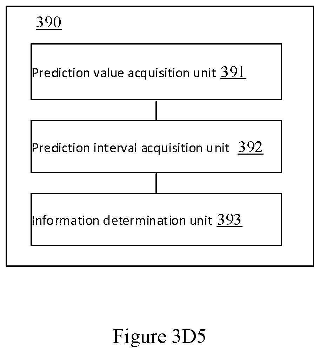

Figure 3D2 is a flowchart illustrating a method for determining human medical information according to an exemplary embodiment;
Figure 3D3A, Figure 3D3B illustrate schematics of vascular segments according to an exemplary embodiment;
Figure 3D4 is a flowchart illustrating a method for training a blood flow prediction model according to an exemplary embodiment;
Figure 3D5 is a schematic diagram of a device for determining human medical information according to an exemplary embodiment;

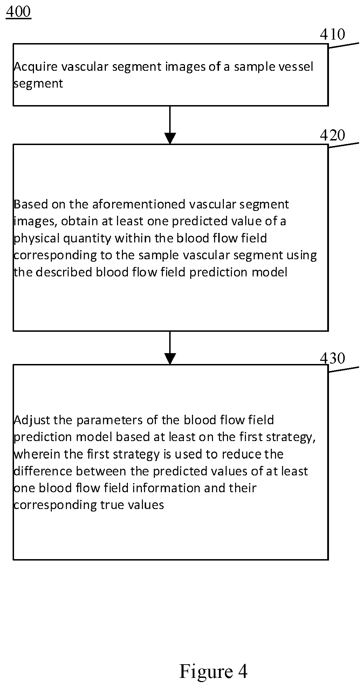

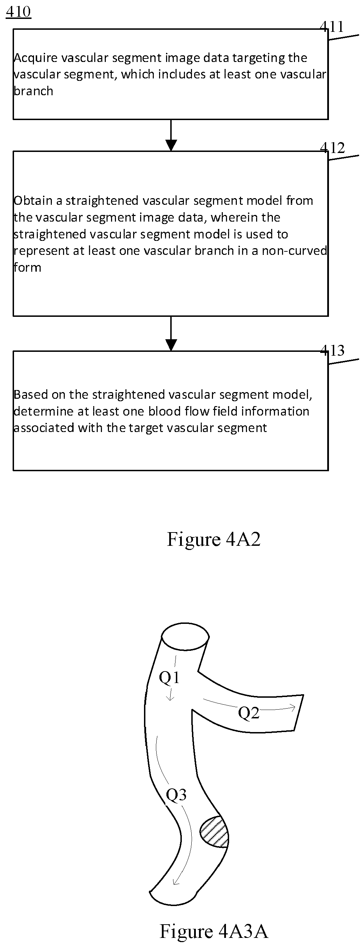

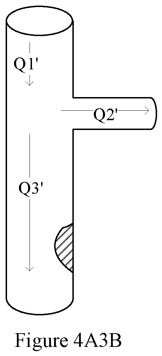

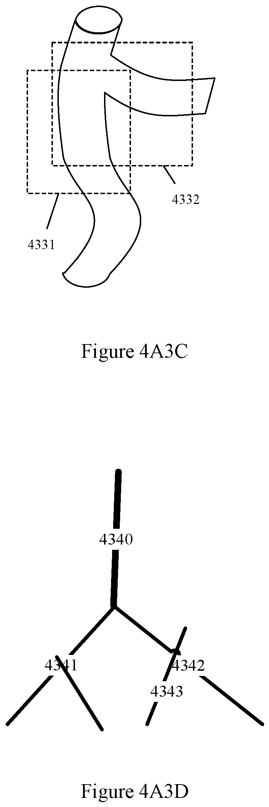

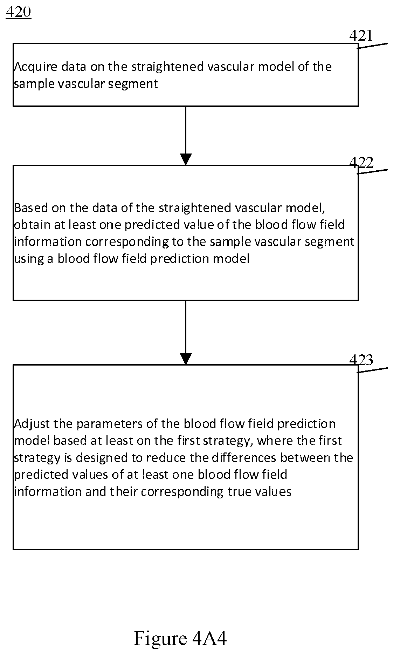

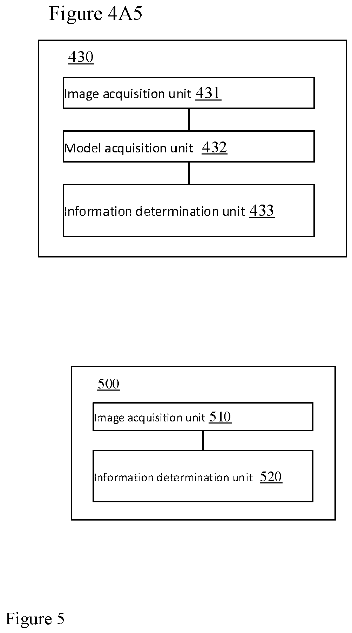

Figure 4 is a flowchart illustrating a method for training a blood flow prediction model according to an exemplary embodiment;
Figure 4A2 is a flowchart illustrating a method for determining blood flow field information according to an exemplary embodiment;
Figure 4A3A, Figure 4A3B, Figure 4A3C and figure 4A3D illustrate schematics of exemplary vascular segments;
Figure 4A4 is a flowchart illustrating a method for training a blood flow prediction model according to an exemplary embodiment;
Figure 4A5 is a schematic diagram of a device for determining blood flow field information according to an exemplary embodiment;
Figure 5 is a schematic diagram of a device for determining blood flow field information according to an exemplary embodiment;

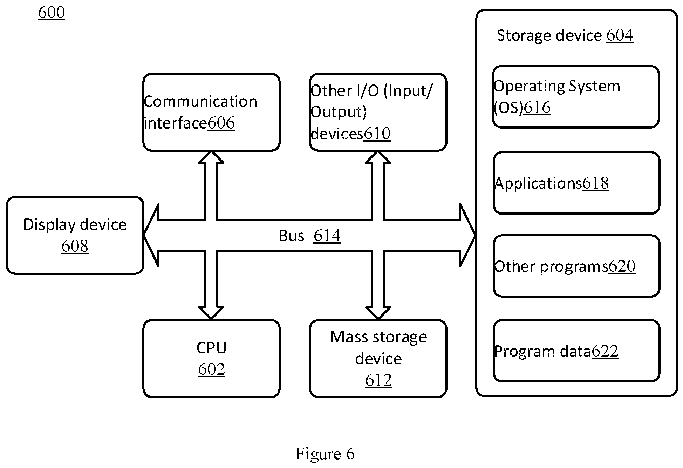

Figure 6 is a diagram of an exemplary computing device that can be applied to exemplary embodiments.

### DESCRIPTION OF THE EMBODIMENTS

In the present disclosure, unless otherwise specified, the use of terms such as "first," "second," etc., to describe various elements is not intended to limit the positional relationship, temporal relationship, or importance relationship of these elements; such terms are only used to distinguish one component from another. In some examples, the first element and the second element may refer to the same instance of that element, while in certain cases, based on contextual description, they may also refer to different instances.

The terms used in the description of various examples in the present disclosure are only for the purpose of describing specific examples and are not intended to be limiting. Unless the context explicitly indicates otherwise, if the number of elements is not intentionally specified, the element may be one or more. As used herein, the term "multiple" refers to two or more, and the term "based on" should be interpreted as "at least partially based on." Additionally, the terms "and/or" and "at least one of" cover any one of the listed items and all possible combinations thereof.

The following describes exemplary embodiments of the present disclosure in detail in conjunction with the accompanying drawings.

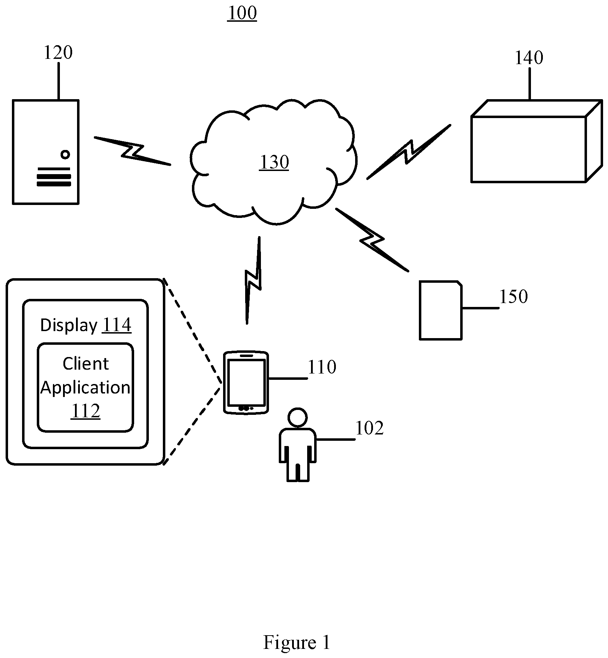

FIG. 1 is a schematic diagram illustrating an exemplary system 100 in which various methods described herein may be implemented according to exemplary embodiments.

Referring to FIG. 1, the system 100 includes a client device 110, a server 120, and a network 130 coupling the client device 110 and the server 120 for communication.

The client device 110 includes a display 114 and a client application (APP) 112 that can be displayed via the display 114. The client application 112 may be an application that needs to be downloaded and installed before running or a lightweight application such as a lite app. In the case where the client application 112 is an application that needs to be downloaded and installed before running, the client application 112 may be pre-installed on the client device 110 and activated. In the case where the client application 112 is a lite app, the user 102 can directly run the client application 112 on the client device 110 by searching for the client application 112 in the host application (e.g., by the name of the client application 112) or scanning the graphic code of the client application 112 (e.g., barcode, QR code, etc.) without installing the client application 112. In some embodiments, the client device 110 can be any type of mobile computing device, including mobile computers, mobile phones, wearable computing devices (such as smartwatches, wearable devices including smart glasses, etc.), or other types of mobile devices. In some embodiments, the client device 110 may alternatively be a fixed computing device, such as a desktop computer, server computer, or other types of fixed computing devices. In some optional embodiments, the client device 110 may also be or include a medical image printing device.

The server 120 is typically deployed by an Internet service provider (ISP) or an Internet content provider (ICP). The server 120 may represent a single server, a cluster of multiple servers, a distributed system, or a cloud server providing basic cloud services (such as cloud databases, cloud computing, cloud storage, cloud communications). It will be understood that although the server 120 is shown in FIG. 1 to communicate with only one client device 110, the server 120 may simultaneously provide backend services to multiple client devices.

Examples of the network 130 include local area networks (LANs), wide area networks (WANs), personal area networks (PANs), and/or combinations of communication networks such as the Internet. The network 130 can be a wired or wireless network. In some embodiments, technologies and/or formats including Hypertext Markup Language (HTML), Extensible Markup Language (XML), etc., may be used to process data exchanged via the network 130. Additionally, encryption technologies such as Secure Socket Layer (SSL), Transport Layer Security (TLS), Virtual Private Network (VPN), Internet Protocol Security (IPsec), etc., may be used to encrypt some or all links. In some embodiments, custom and/or proprietary data communication technologies may be used to replace or supplement the above data communication technologies.

The system 100 may also include an image capture device 140. In some embodiments, the image capture device 140 shown in FIG. 1 may be a medical scanning device, including but not limited to positron emission tomography (PET), positron emission tomography with computerized tomography (PET/CT), single photon emission computed tomography with computerized tomography (SPECT/CT), computed tomography (CT), medical ultrasonography, nuclear magnetic resonance imaging (NMRI), magnetic resonance imaging (MRI), cardiac angiography (CA), digital radiography (DR), and other scanning or imaging devices used in medical imaging. For example, the image capture device 140 may include a digital subtraction angiography scanner, a magnetic resonance angiography scanner, a tomographic angiography scanner, a positron emission tomography scanner, a positron emission tomography with computerized tomography scanner, a single photon emission computed tomography scanner, a computed tomography scanner, a medical ultrasonography device, a nuclear magnetic resonance imaging scanner, a magnetic resonance imaging scanner, a digital radiography scanner, etc. The image capture device 140 may be connected to a server (e.g., server 120 shown in FIG. 1 or a separate server not shown in the figure of the imaging system) to process image data, including but not limited to converting scan data (e.g., into medical image sequences), compression, pixel correction, three-dimensional reconstruction, etc.

The image capture device 140 may be connected to the client device 110 via the network 130 in some embodiments, or may be directly connected to the client device for communication in other ways.

Optionally, the system may also include an intelligent computing device or computing card 150. The image capture device 140 may include or be connected (e.g., removably connected) to such computing card 150, etc. As an example, the computing card 150 may perform processing of image data, including but not limited to conversion, compression, pixel correction, reconstruction, etc. As another example, the computing card 150 may implement methods for determining blood flow field information according to exemplary embodiments of the present disclosure.

The system may also include other unillustrated components, such as a data storage unit. The data storage unit may be a database, data repository, or one or more devices used for data storage in other forms, which may be a conventional database, or may include cloud databases, distributed databases, etc. For example, directly captured image data formed by the image capture device 140 or medical image sequences or three-dimensional image data obtained through image processing may be stored in the data storage unit for subsequent retrieval by the server 120 and the client device 110 from the data storage unit. Additionally, the aforementioned image capture device 140 may directly provide image data or medical image sequences or three-dimensional image data obtained through image processing to the server 120 or the client device 110, etc.

Users may use the client device 110 to control the capture of images or medical images, view captured images or images (including preliminary image data or analyzed images, etc.), view analysis results, interact with captured images or analysis results, input capture instructions, configure data, etc. The client device 110 may send configuration data, instructions, or other information to the
image capture device 140 to control image capture, data processing, etc.

For the purposes of exemplary embodiments of the present disclosure, in the example shown in FIG. 1, the client application 112 can be an image sequence management application, which can provide various functions such as storage management, indexing, sorting, classification, etc., for the captured image sequences. Correspondingly, the server 120 can be a server used in conjunction with the image sequence management application. The server 120 can provide image sequence management services to the client device 110 running the client application 112 based on user requests or instructions generated according to exemplary embodiments of the present disclosure, such as managing cloud-based image sequence storage, storing and classifying image sequences according to specified indices (including but not limited to sequence type, patient identifier, body part, target of acquisition, acquisition stage, acquisition machine, presence of lesion detection, severity, etc.), and retrieving and providing image sequences to the client device based on specified indices, etc. Alternatively, the server 120 may also provide or allocate such service capabilities or storage space to the client device 110, and the client application 112 running on the client device 110 may provide corresponding image sequence management services based on user requests or instructions generated according to exemplary embodiments of the present disclosure, etc. It will be understood that the above is merely an example, and the present disclosure is not limited thereto.

FIG. 2 is a flowchart illustrating a method 200 for determining blood flow field information according to exemplary embodiments. The method 200 may be executed on a client device (e.g., client device 110 shown in FIG. 1), i.e., the entity executing the various steps of the method 200 may be the client device 110 shown in FIG. 1. In some embodiments, the method 200 may be executed on a server (e.g., server 120 shown in FIG. 1). In some embodiments, the method 200 may be executed by a combination of a client device (e.g., client device 110) and a server (e.g., server 120).

In the following, each step of the method 200 is described in detail.

Referring to FIG. 2, at step 210, a vascular segment image of a target blood vessel segment is obtained.

At step 220, based on the vascular segment image, at least one blood flow field information for the target blood vessel segment is determined through a pre-trained neural network.

Through the above method, blood flow field information can be directly obtained based on the vascular segment image.

Referring to FIG. 3A, a data flow according to exemplary embodiments of the present disclosure is illustrated. For example, images or image sequences collected about the target vascular segment can be input into the network, enabling the network to output flow field information. Throughout this disclosure, the trained neural network may be referred to as a flow field information determination network, a flow field information determination model, a blood information determination model, etc., and the present disclosure is not limited thereto.

Various types of vascular segment images can be used. By way of example, vascular segment images may be CT images. According to some embodiments, the vascular segment image may be images collected for the target vascular segment using the digital subtraction angiography (DSA) method. By way of example, the vascular segment image for the target vascular segment may be coronary CTA images collected using computed tomography angiography.

In diagnosing coronary artery disease, it is often necessary to assess the health of the coronary arteries. For example, functional assessment can be made based on the fractional flow reserve (FFR). FFR refers to the ratio of the maximum blood flow that can be obtained in a myocardial area supplied by a coronary artery in the presence of stenotic lesions in the coronary artery to the maximum blood flow that can theoretically be obtained in the same area under normal conditions, i.e., the ratio of the mean pressure in the distal coronary artery during maximum myocardial perfusion (Pd) to the mean pressure in the proximal coronary artery (Pa). According to some embodiments, the human medical information includes FFR information.

According to some embodiments, the method may further include determining at least one type of blood flow field information based on the analysis purpose, where the analysis purpose indicates the category of human medical information to be obtained based on the vascular segment image, and wherein the determination of at least one blood flow field information for the target blood vessel segment is based on the at least one type of blood flow field information.

According to some embodiments, the determined at least one type of blood flow field information corresponds to the category of human medical information. For example, in the example continued from above, when the category of human medical information to be obtained is FFR, blood flow field information including pressure P, such as pressure values at corresponding locations, can be determined.

According to some embodiments, the determined at least one type of blood flow field information includes a first type of blood flow field information and a second type of blood flow field information, wherein the first type of blood flow field information is used to generate the category of human medical information, and the second type of blood flow field information can meet one or more physical constraint relationships with respect to the first type of blood flow field information.

As a specific non-limiting example, in the case where the desired human medical information includes FFR values, in addition to information about pressure P, predictions can be made for variables such as velocity v, flow rate Q, and/or viscosity η, and these predicted physical quantities can also be made to satisfy physical constraints, such as one or more fluid mechanics equations or dynamic equations. Such prediction models can predict more comprehensive physical quantities, thus providing a deeper understanding of physical relationships and therefore greater accuracy. In other words, the method may include obtaining predictions of at least one first type of physical quantity and at least one second type of physical quantity for the blood flow field corresponding to the target vascular segment based on the vascular segment image, wherein the predictions of the at least one first type of physical quantity and the at least one second type of physical quantity satisfy at least one constraint condition. The first type of physical quantity may correspond to the first type of blood flow field information, and the second type of physical quantity may correspond to the second type of blood flow field information. The predictions of the second type of physical quantity may not be used to determine human medical information, but only to supervise the first type of physical quantity and/or the model itself based on physical constraint conditions.

Exemplary constraint conditions may be physical constraint conditions. For example, physical quantities that satisfy the constraint conditions may satisfy one or more physical equations or physical constraints expressed in other forms. It will be understood that "satisfying" constraint conditions may mean that the calculated value based on the predicted values of the corresponding physical quantities falls within a predetermined threshold range of the standard formula, and the present disclosure is not limited thereto.

As another non-limiting example, the categories of human medical information can include plaque risk assessment, stent surgery guidance information, and so forth. Illustratively, in the case where the category of human medical information is plaque risk assessment, it is necessary to assess the degree or risk of plaque rupture. In such a scenario, the types of blood flow field information can be determined as velocity (v) and wall shear stress, to base the determination of corresponding blood flow field information on this. Optionally, the aforementioned velocity (v) and wall shear stress can be designated as the first type of flow field information (first physical quantity), and additionally, the second type of flow field information (second physical quantity) can be determined, such as including pressure (P), flow rate (Q), etc., and the model can further generate predictions for these second physical quantities, ensuring that the first and second physical quantities satisfy one or more physical constraint relationships, thereby ensuring the model's output is equivalent to physical formulas, meeting the rationality of the output results from a physical perspective.

As another specific example, in the case where the category of human medical information is stent surgery guidance information, the types of blood flow field information can be determined as pressure (P) and velocity (v), to generate corresponding human medical information based on predicted blood flow field information. Optionally, the aforementioned pressure (P) and velocity (v) can be designated as the first type of flow field information (first physical quantity), and additionally, the second type of flow field information (second physical quantity) can be determined, such as including flow rate (Q), viscosity ( η ), etc., and this disclosure is not limited thereto.

Continuing from the previous example, a "design" scheme based on the analysis purpose can be provided according to one or more embodiments of the present disclosure. For example, a corresponding set of physical quantities or combinations of physical quantities to be predicted can be designed based on the corresponding analysis purpose (e.g., FFR, plaque risk assessment, etc.). By designing the types of output blood flow field information, the optimal solution for the current analysis purpose can be selected, taking into account the current analysis target and physical interpretability, enhancing the model's output. For example, in the case where more second types of flow field information ("additional physical quantities") are constrained to increase the rationality of the model.

In some embodiments, the constraint conditions can be physical constraint conditions. For example, physical quantities that satisfy the constraint conditions can meet one or more physical formulas or constraints represented in other forms. It should be understood that "satisfying" the constraint conditions can be within a predetermined threshold range based on the difference between the calculated value of the predicted value of the corresponding physical quantity and the standard formula, and the present disclosure is not limited thereto.

In some embodiments, the first physical quantity and the second physical quantity can be predicted by a single model. In such embodiments, because multiple physical quantities predicted by a single model satisfy constraint conditions, such a model can have a deeper understanding of the physical quantities. For example, the predicted values of the first physical quantity and the second physical quantity can be obtained through a pre-trained neural network. In such examples, the network itself can not only learn individual physical quantity values but also learn the relationship between physical quantities, making it more accurate and efficient. In other embodiments, multiple models can also be used for prediction separately.

According to one or more embodiments of the present disclosure, the effectiveness of the model can be made equivalent to physical formulas, ensuring that the model's output is close to physical formulas for each physical quantity, thereby ensuring the rationality from a physical perspective. For example, in the process of extracting physical parameters from medical images, at least two physical quantities can be predicted (for example, in addition to generating physical quantities required for medical information, additional physical quantities can be predicted), and the predicted physical quantities can have physical fitting ability, ensuring the rationality of the model's output.

In some embodiments, the blood flow field can be a pressure field. In some embodiments, the first physical quantity can be pressure, and the second physical quantity can include at least one of velocity and flow rate. Thus, important physical quantities in the blood flow field can be obtained, and other physiological or pathological information related to the target blood vessel can be determined accordingly.

As a further non-limiting example, the human medical information may include blood flow reserve score (FFR) information. When diagnosing coronary artery disease, it is often necessary to assess the health of the coronary arteries. Illustratively, functional assessments based on fractional flow reserve (FFR) can be made. FFR refers to the ratio of the maximum blood flow that can be obtained in the myocardial area supplied by the coronary artery when stenotic lesions exist in the coronary artery to the maximum blood flow that can theoretically be obtained in the same area under normal conditions, i.e., the ratio of the
mean pressure (Pd) in the distal coronary artery during maximal myocardial hyperemia to the mean pressure (Pa) in the aortic root at the entrance of the coronary artery. It can be understood that the predicted pressure values can be used to calculate the FFR value.

Continuing from the previous example, for instance, in the case where the human medical information includes plaque risk assessment, the first physical quantity can include velocity (v) and wall shear stress, and the second physical quantity can include pressure (P), flow rate (Q), etc. In the case where the human medical information includes stent surgery guidance information, the first physical quantity can include pressure (P) and velocity (v), and the second physical quantity can include flow rate (Q), viscosity ( η ), etc., and this disclosure is not limited thereto.

Continuing from the previous text regarding FFR prediction, for example, with knowledge of the location of lesions, the model can predict pressure field information for the upstream and downstream regions of the lesion (e.g., arterial stenosis), thereby obtaining more accurate estimates without wasting computational resources.

In some embodiments, the method may also include obtaining a prediction interval for at least one predicted physical quantity, and wherein at least one human medical information regarding the target blood vessel segment can be based on the predicted physical quantity and the prediction interval. For example, when calculating human medical information, referencing the predicted interval can reduce introduced errors and improve calculation accuracy.

Exampled, the method may further include obtaining updated predicted values of corresponding physical quantities of the blood flow field information based on the predicted interval, and at least one human medical information can be determined based on the updated predicted values. In such embodiments, the predicted values of the blood flow field information (e.g., a portion of the blood flow field information) can be updated based on the predicted interval, and the human medical information can be determined based on the updated predicted values, thereby increasing the prediction accuracy. Due to the complex structure of the human body and the conditions of image acquisition, images of vascular segments often have occlusion, distortion, overlap, etc., which may result in differences in image clarity, accuracy, and angiographic imaging between some vascular segments and others. Alternatively, certain vessels may be more distorted, fluctuate more with respiration and heartbeats, be smaller, or have blurry boundaries, which may lead to errors in the model's prediction capabilities. For example, the predicted values and prediction intervals of better vascular segments with clearer imaging and more stable morphology can be used, and the predicted values of poorer vascular segments with less clear imaging, occlusion, and larger fluctuations over time can be updated using the relationship between them. For instance, the target vascular segment may include a first vascular segment connected to a second vascular segment via a branching point. For example, one of the first and second vascular segments can be a side branch vessel, while the other is a main vessel. Alternatively, both the first and second vascular segments can be side branch vessels. In other words, the updated predicted values of corresponding physical quantities of the blood flow field information based on the predicted interval can include the predicted interval of corresponding physical quantities of the blood flow field information at at least one location in the first vascular segment, and the updated predicted values of corresponding physical quantities of the blood flow field information at at least one location in the second vascular segment can be determined.

Additionally, it can be understood that obtaining prediction intervals associated with the predicted values may include determining predicted values and/or prediction intervals of corresponding physical quantities of the blood flow field information at one or more positions (e.g., one or more prediction points) in the first vascular segment. Such positions or prediction points may be sampled or selected based on predetermined or machine-automated resolutions, or additionally based on the locations of lesions, lesion positions, key point positions, etc., and the present disclosure is not limited thereto.

As another example of updating predicted values of associated vessels based on vascular topology, in the target vascular segment, there may exist a first vascular segment and a second vascular segment connected to a third vascular segment via the branching point. For example, one of the first, second, and third vascular segments can be a main vessel, while the others can be side branch vessels. The main vessels in the first, second, and third vascular segments may branch into two side branch vessels, or may be formed by the convergence of two side branch vessels. Alternatively, both the first, second, and third vascular segments can be main vessels, with one of them being a side branch vessel. In such examples, the updated predicted values of corresponding physical quantities of the blood flow field information at at least one location in the second vascular segment can be based on the predicted interval of corresponding physical quantities of the blood flow field information at at least one location in the third vascular segment.

Exemplarily, determining the updated predicted values of corresponding physical quantities of the blood flow field information at at least one location in the second vascular segment based on the predicted interval of corresponding physical quantities of the blood flow field information at at least one location in the first vascular segment can include: based on the blood flow convergence relationship at the branching point, and based on the predicted interval of corresponding physical quantities of the blood flow field information at at least one location in the first vascular segment and the predicted interval of corresponding physical quantities of the blood flow field information at at least one location in the third vascular segment, determining the updated predicted values of corresponding physical quantities of the blood flow field information at at least one location in the second vascular segment. The blood flow convergence relationship may include conservation or equation relationships composed of blood inflow and outflow, such as the conservation of blood flow between inflow and outflow branches. For example, at adjacent or nearly adjacent positions in different vascular segments connected to the branching point, the predicted pressure values should be roughly equal, continuous, or otherwise meet physical constraint conditions. Exemplarily, each prediction interval may include upper and lower bounds, and updating the predicted values may include adjusting the predicted values or prediction intervals to satisfy corresponding conservation or equation relationships between the connected vascular segments.

Additionally, it can be understood that throughout this disclosure, the model described for training methods can be applied to prediction methods or determination methods in other embodiments of this disclosure, and the prediction methods or determination methods in embodiments of this disclosure can use models trained according to the training methods in embodiments of this disclosure, or other models or algorithms understood by those skilled in the art. For brevity, these repeated or similar features are not repeated herein.

Furthermore, it can be understood that although the operations are depicted in the accompanying drawings as being performed in a specific order, this should not be construed as requiring the operations to be performed in the order shown or in sequential order, nor should it be construed as requiring all the operations shown to be performed to achieve the desired result. For example, the two steps described in order in this document can be performed in reverse order, or concurrently. Similarly, one or more steps may be omitted in various embodiments of this disclosure.

Moreover, it can be understood that the methods for predicting or determining data described in embodiments of this disclosure are not methods for directly determining diagnostic results by doctors, but involve data processing or information processing processes in medical processes, whose data processing results can be used for reference by doctors to assist in their medical operations. It can be understood that the information processing methods, data prediction methods, determination methods, decision methods, etc., described in embodiments of this disclosure are executed by computers or devices containing computers.

It can also be understood that throughout this disclosure, images, image data, or image sequences may include or consist of two-dimensional image data, or may include or consist of three-dimensional image data. Images, image data, or image sequences may be directly acquired and stored or otherwise transmitted to terminal devices for user use. Images, image data, or image sequences may also be processed after various image processing processes. Images, image data, or image sequences may undergo other analysis processes (e.g., lesion feature analysis) and may contain analysis results (e.g., circled regions of interest, segmentation results of tissues, etc.). It can be understood that this disclosure is not limited thereto.

Exemplarily, the device for determining human medical information 320 according to exemplary embodiments may include an image acquisition unit 321, a physical quantity prediction unit 322, and an information determination unit 323. The image acquisition unit 321 may be used to obtain vascular segment image data about the target vascular segment. The physical quantity prediction unit 322 may be used to obtain predicted values of a first physical quantity and a second physical quantity in the blood flow field corresponding to the target vascular segment based on the vascular segment image data, where the predicted values of the first physical quantity and the second physical quantity satisfy at least one physical constraint condition. The information determination unit 323 may be used to determine at least one human medical information regarding the target vascular segment based on the predicted value of the first physical quantity.

It should be understood that the various modules of the device 320 shown in Figure 3A4 can correspond to the various steps of the method 300 described with reference to Figure 3A2. Thus, the operations, features, and advantages described above for method 300 and its variant examples apply equally to device 320 and its included modules. For brevity, certain operations, features, and advantages are not reiterated here.

Figure 3A5 is a schematic diagram illustrating a training device 330 for the blood flow prediction model according to exemplary embodiments. The training device 330 for the blood flow prediction model may include: an image acquisition unit 331, a physical quantity prediction unit 332, and a model adjustment unit 333. The image acquisition unit 331 may be used to obtain vascular segment image data about a sample vascular segment. The physical quantity prediction unit 332 may be used to obtain, based on the vascular segment image data, predictions of a first physical quantity and a second physical quantity in the blood flow corresponding to the sample vascular segment through the blood flow prediction model, where the predictions of the first physical quantity and the second physical quantity satisfy at least one physical constraint condition. The model adjustment unit 333 may be used to adjust parameters of the blood flow prediction model based on at least one of the first strategy, the second strategy, and the third strategy, where the first strategy is used to reduce the difference between the prediction of the first physical quantity and the true value of the first physical quantity, the second strategy is used to reduce the difference between the prediction of the second physical quantity and the true value of the second physical quantity, and the third strategy is used to make the predictions of the first physical quantity and the second physical quantity satisfy at least one physical constraint condition.

It should be understood that the various modules of the device 330 shown in Figure 3A5 can correspond to the various steps of the method 310 described with reference to Figure 3A3. Thus, the operations, features, and advantages described above for method 310 and its variant examples apply equally to device 330 and its included modules. For brevity, certain operations, features, and advantages are not reiterated here.

According to exemplary embodiments disclosed herein, a computing device is also disclosed, comprising a memory, a processor, and a computer program stored in the memory, wherein the processor is configured to execute the computer program to implement at least one of the steps of the method for determining human medical information and the training method for the blood flow prediction model according to exemplary embodiments disclosed herein and its variant examples.

According to exemplary embodiments disclosed herein, a non-transitory computer-readable storage medium is also disclosed, on which computer program is stored, wherein when the computer program is executed by a processor, the processor implements at least one of the steps of the method for determining human medical information and the training method for the blood flow prediction model according to exemplary embodiments disclosed herein and its variant examples.

According to one aspect disclosed herein, a computer program product is provided, comprising a computer program that, when executed by a processor, implements steps of any one of the method embodiments described above.

While specific modules have been discussed above with reference to specific functionalities, it should be noted that the functionalities of the various modules discussed herein can be divided among multiple modules, and/or at least some functionalities of multiple modules can be combined into a single module. The actions performed by specific modules as discussed herein include the specific module itself performing the action, or alternatively, the specific module calling or otherwise accessing another component or module to perform the action (or performing the action together with the specific module). Thus, the specific module performing the action can include the specific module itself performing the action and/or another module accessed by or accessed by the specific module performing the action. For example, in one or more embodiments described herein, various modules or units may be combined into a single module or unit. Additionally, in one or more embodiments described herein, two or more modules or units may be described in parallel, while in other embodiments, these modules and units may have one or more containment relationships. As used herein, the phrase "entity A initiates action B" or "entity A causes action B" may refer to entity A issuing instructions to perform action B, but entity A itself may not necessarily perform action B.

As a specific non-limiting example, referring to Figure 3B, lesion information (e.g., lesion location) can be inputted as prompt information into the model used to determine blood flow field information. As another non-limiting example, referring to Figure 3C, the model can be trained to additionally possess the ability to predict lesion information, thereby deepening the understanding of the current image.

In some embodiments, the lesion information may include segmentation results related to at least one lesion area. As additional or alternative examples, lesion information may include lesion location, size, type, severity, and so forth. For example, using lesion information as a reference can involve personalizing various thresholds related to the current image acquisition, imaging environment, contrast agent, imaging level, with the help of lesion segmentation. For instance, it could involve calibrating the overall image intensity level based on the CT values (e.g., pixel color depth) of lesion and non-lesion areas. Additionally, using lesion information as a reference can involve higher accuracy, precision, computational power, and lower error threshold requirements for flow field predictions at lesion sites. Furthermore, using lesion information as a reference can include determining sampling points, prediction points, key points, etc., based on lesion location. For example, the density of points for generating flow field information can be determined based on lesion size, ensuring that the scale of the resulting output matches the lesion size. Likewise, key points can be determined based on lesion type, location, etc., where specific flow field information at these key points holds higher importance for subsequent diagnosis and treatment. Continuing the example regarding FFR prediction from earlier, with knowledge of the lesion location, the model can predict pressure field information upstream and downstream of the lesion area (e.g., arterial stenosis) with higher precision, thus obtaining more accurate estimates without wasting computational resources.

In some embodiments, the blood flow field can be a pressure field. For instance, the determined blood flow field information may include at least one of pressure, velocity, and flow rate. Thus, important physical quantities within the blood flow field can be obtained, which can then be used to determine other physiological or pathological information related to the target vessel segment. In some embodiments, the method may also include determining at least one human medical information regarding the target vessel segment based on at least one blood flow field information.

In such embodiments, the model output can conform to physical formulas, ensuring the accuracy and reasonableness of the model output at the physical level.

In some embodiments, the method may further include obtaining prediction intervals for at least one blood flow field information regarding the target vessel segment, and wherein at least one human medical information about the target vessel segment can also be based on the prediction intervals. For example, updated predicted values for the blood flow field information can be obtained based on the prediction intervals, and at least one human medical information can be determined based on the updated predicted values. In such embodiments, the prediction intervals can be used to update predicted values for the blood flow field information (e.g., a portion of the blood flow field information), and human medical information can be determined based on the updated predicted values, thereby increasing prediction accuracy.

Due to the complex structure of the human body and imaging conditions, vascular images often exhibit occlusions, distortions, overlaps, etc., leading to differences in image clarity, accuracy, and contrast enhancement for some vascular segments compared to others. Alternatively, models may have a certain degree of error in predicting vascular segments that are more twisted, have larger morphological fluctuations due to respiration and heartbeat, are smaller in size, or have blurred boundaries. For example, predicted values and prediction intervals for superior vascular segments with clearer imaging and more stable morphology can be used to update predicted values for inferior vascular segments with less clear imaging, occlusions, greater temporal fluctuations, etc. For example, the target vascular segment may include a first vascular segment and a second vascular segment, where the first vascular segment connects to the second vascular segment via a branching point. For instance, one of the first and second vascular segments may be a side branch while the other is a main vessel. Alternatively, both the first and second vascular segments may be side branches. In such cases, updated predicted values for blood flow field information can be determined based on prediction intervals for at least one position in the first vascular segment, and updated predicted values for blood flow field information can be determined for at least one position in the second vascular segment.

Furthermore, it can be understood that obtaining prediction intervals associated with the predicted values can include determining predicted values and/or prediction intervals for blood flow field information at one or more positions (e.g., one or more prediction points) in the first vascular segment, respectively. Such positions or prediction points may be sampled or selected based on predefined or machine-selected resolutions, or additionally based on lesion locations, lesion positions, key point locations, and so forth. The disclosure is not limited to this.

As another example of updating predictions for associated vessels based on vascular topology, in the target vascular segment, there may exist a scenario where a first vascular segment and a second vascular segment are connected to a third vascular segment via branching points. For example, one of the first, second, and third vascular segments may
be a main vessel, and the others may be side branches. The main vessels in the first, second, and third vascular segments may branch into two side branches, or may converge from two side branches. Alternatively, both the first and second vascular segments may be main vessels, with the third being a side branch. In such examples, updated predicted values for at least one position in the second vascular segment can be based on prediction intervals for blood flow field information at at least one position in the third vascular segment.

For example, based on prediction intervals for blood flow field information at at least one position in the first vascular segment, updated predicted values for at least one position in the second vascular segment can include: based on blood flow convergence relationships at the branching points, and based on prediction intervals for blood flow field information at at least one position in the first vascular segment and at least one position in the third vascular segment, determining updated predicted values for at least one position in the second vascular segment. Blood flow convergence relationships can include conservation or equation relationships formed by the inflow and outflow of blood, such as conservation of blood flow between inflow and outflow branching points. For example, predicted intervals can include upper and lower prediction limits, and updating predicted values can include adjusting predicted values or prediction intervals to satisfy corresponding conservation or equation relationships between interconnected vascular segments.

The schematic diagrams described in Figure 3A, Figure 3B, and Figure 3C illustrate data flows according to some exemplary embodiments of the present disclosure. As shown in Figure 3A, in some embodiments, at least one blood flow field information associated with the target vascular segment can be determined through a pre-trained neural network, which can also be referred to as a blood flow field information determination network or model, or simply as a flow field determination model or network. For example, image data can be inputted into the flow field determination network, allowing the network to output flow field results.

Referring to Figure 3B, it illustrates, according to a specific non-limiting embodiment of the present disclosure, wherein lesion information including lesion location can be input as prompt information into the blood flow field determination network. By way of example, lesion information may include segmentation results such as segmentation masks, segmentation matrices, and so on, and the present disclosure is not limited thereto. Additionally, lesion information may include the type and severity of the lesion. Examples include using additional models different from the blood flow field determination network, such as lesion analysis models, lesion segmentation models, lesion localization models, lesion grading determination models, and so on, to obtain lesion information, and the present disclosure is not limited thereto.

Referring to Figure 3B, it further illustrates, according to another specific non-limiting embodiment of the present disclosure, wherein lesion information associated with at least one lesion area related to the target vessel segment can also be obtained through the blood flow field determination network or blood flow field determination model. In such embodiments, at least one blood flow field information associated with the target vessel segment may be determined by a pre-trained neural network, and obtaining lesion information associated with at least one lesion area related to the target vessel segment may include obtaining lesion information through the neural network. In such embodiments, the flow field determination model itself can also learn the understanding of lesions, thereby making the network's understanding of image data more comprehensive and helping to guide the flow field results themselves.

By way of example, using lesion information as a reference can include personalizing various thresholds related to the current image acquisition, imaging environment, contrast agent, imaging level with the help of lesion segmentation. For instance, the overall image intensity level can be calibrated based on the CT values (e.g., pixel color depth) of lesion and non-lesion areas. As another example, using lesion information as a reference can involve higher accuracy, precision, computational power, and lower error threshold requirements for flow field predictions. As additional or alternative examples, using lesion information as a reference can involve determining sampling points, prediction points, key points, etc., based on lesion location. For example, the density of points for generating flow field information can be determined based on lesion size, ensuring that the resulting output scale matches the lesion size. Similarly, key points can be determined based on lesion type, location, etc., where specific flow field information at these key points holds higher importance for subsequent diagnosis, treatment, and so forth. Continuing the example regarding FFR prediction, with knowledge of the lesion location, the model can predict pressure field information upstream and downstream of the lesion area with higher precision, thus obtaining more accurate estimates without wasting computational resources.

It can be understood that the models or networks required in one or more embodiments of the present disclosure can be trained in any manner understood by those skilled in the art, such as a model or network for predicting blood flow field information. For ease of understanding, a non-limiting exemplary training method 350 is described in conjunction with Figure 3B4.

At step 351, data regarding the sample vessel segment is obtained.

At step 352, based on the data, at least one blood flow field information associated with the sample vessel segment is obtained through the blood flow field prediction model.

At step 353, the parameters of the blood flow field prediction model are adjusted based on at least a first strategy, where the first strategy is used to reduce the difference between the at least one blood flow field information and the corresponding ground truth.

In some exemplary embodiments, such as the embodiment described in reference to Figure 3B, the data regarding the sample vessel segment may include vessel segment image data and lesion information (e.g., lesion location, lesion size, lesion type, etc.). For example, step 352 may include obtaining at least one blood flow field information associated with the sample vessel segment based on the collected (or processed) vessel segment image data and lesion information through the blood flow field prediction model.

In other exemplary embodiments, such as the example described in reference to Figure 3C, the data regarding the sample vessel segment may include only vessel segment image data, and the trained network or model may also output lesion information (e.g., lesion location, lesion size, lesion type, etc.). For example, step 353 may further include adjusting the parameters of the blood flow field prediction model based on a second strategy, where the second strategy is used to reduce the difference between the predicted lesion information and the corresponding ground truth.

By way of example, the at least one blood flow field information can be used to determine at least one human medical information regarding the sample vessel segment.

Additionally, the parameters of the blood flow field prediction model can be adjusted based on additional strategies to ensure that the various physical quantities of the at least one blood flow field information satisfy at least one constraint. By way of example, the at least one constraint can be a physical constraint. For instance, physical constraints can be equations or other representations of physical-level constraints formed by one or more physical formulas. As a specific non-limiting example, the true value of flow rate Q can be estimated based on the true values of pressure P and velocity v through a physical equation, and the predicted values of pressure, velocity, and flow rate can be compared with their corresponding true values, and if they match, it can be considered to satisfy the physical constraint.

According to some embodiments, the vessel segment image data can be image data processed by straightening, where the straightening process is used to straighten one or more curved vessel branches of the target vessel segment into a non-curved vessel branch model. For example, the processed image data can be straightened images (or sequences of straightened images), where one or more curved vessel branches of the target vessel segment are straightened. The processed image data can be or represented as a three-dimensional model. Since the flow field information of interest in medical imaging modeling is often known flow field information for specific points, straightening the curved vessels into straightened vessels in this case does not introduce measurable errors into the calculation results; however, it greatly simplifies the model complexity of the vessels, thereby simplifying the processing of modeling and calculation, and increasing computational efficiency.

According to some embodiments, obtaining the straightened vessel segment model based on the vessel segment image data may include: obtaining the vessel centerline of at least one vessel branch based on the vessel segment image data; and modeling based on the vessel centerline to obtain the straightened vessel segment model. For example, the vessel centerline of the curved vessel can be obtained first, and then it can be unfolded and deformed into a linear form (such as a cylinder, approximate cylinder, frustum, or cone, etc.) in the length dimension to obtain the straightened vessel segment model. According to some embodiments, modeling based on the vessel centerline to obtain the straightened vessel segment model may include: obtaining vessel cross-sectional data corresponding to multiple points on the vessel centerline; and based on the vessel cross-sectional data and the vessel centerline, obtaining the straightened vessel segment model. For example, multiple points can be obtained based on fixed interval sampling, predetermined multiple sampling points, key points, bifurcation points, etc., and vessel cross-sectional data corresponding to these points can be obtained, and the corresponding cross-sectional data can be mapped onto the unfolded centerline to achieve vessel modeling in a straightened form. According to other embodiments, transformation (straightening process) from curved vessel segment data to straightened vessel segment data can be achieved through pre-trained models. It can be understood that the above
is only exemplary, and other vessel model transformation algorithms that can be understood by those skilled in the art or may appear in the future can also be used to simplify calculations, and the present disclosure is not limited thereto.

By way of example, the method may further include obtaining prediction intervals for the at least one blood flow field information, where the prediction intervals can be used to determine at least one human medical information regarding the target vessel segment. By way of example, obtaining the prediction intervals may include obtaining updated predicted values for the blood flow field information based on the prediction intervals, and at least one human medical information can be determined based on the updated predicted values. In such embodiments, the prediction intervals can be used to update the predicted values of the blood flow field information (e.g., a portion of the blood flow field information), and human medical information can be determined based on the updated predicted values, thereby increasing the accuracy of predictions.

Due to the complex structure of the human body and the limitations of image acquisition conditions, vessel images often suffer from occlusion, distortion, overlap, etc., resulting in differences in image clarity, accuracy, and contrast imaging degrees for some vessel segments compared to others. Alternatively, some vessels may have shapes that are more twisted, fluctuate greatly with the body's breathing and heartbeat, are smaller in size, or have blurry boundaries, leading to a certain degree of error in the model's predictive capability. For example, using predictions and prediction intervals for better vessel segments, such as those with clearer imaging and more stable morphology, to update predictions for poorer vessel segments with less clear imaging, occlusion, or greater fluctuations over time. For instance, the target vessel segment can include a first vessel segment and a second vessel segment, where the first vessel segment can be connected to the second vessel segment via a branching point. For example, one of the first and second vessel segments may be a side branch, and the other may be a main vessel. Alternatively, both the first and second vessel segments can be side branches. In other words, based on the prediction intervals, determining the updated predicted values for the blood flow field information may include determining the updated predicted values for the blood flow field information at at least one position in the second vessel segment based on the prediction intervals determined for at least one position in the first vessel segment.

Furthermore, it can be understood that obtaining prediction intervals associated with the predicted values may include determining predicted values and/or prediction intervals for the blood flow field information at one or more positions (e.g., one or more prediction points) in the first vessel segment. Such at least one position or prediction point can be sampled or selected based on a predetermined or machine-automated resolution, or additionally based on lesion location, lesion position, key point position, and so on, and the present disclosure is not limited thereto.

As another example of updating predicted values for associated vessels based on vascular topology, in the target vessel segment, there can be a scenario where a first vessel segment and a second vessel segment are connected to a third vessel segment via a branching point. For example, among the first vessel segment, the second vessel segment, and the third vessel segment, one may be a main vessel, and the others may be side branches. The main vessel in the first, second, or third vessel segment may branch into two side branches, or may be formed by the convergence of two side branches. Alternatively, both the first and second vessel segments, or both the second and third vessel segments, can be main vessels, with the remaining segment being a side branch. In such examples, the updated predicted values for the blood flow field information at at least one position in the second vessel segment can be based on the prediction intervals for the blood flow field information at at least one position in the third vessel segment.

Exemplarily, determining the updated predicted values for the blood flow field information at at least one position in the second vessel segment based on the prediction intervals for the blood flow field information at at least one position in the first vessel segment may include: based on the blood flow convergence relationship at the branching point, and based on the prediction intervals for the blood flow field information at at least one position in the first vessel segment and the prediction intervals for the blood flow field information at at least one position in the third vessel segment, determining the updated predicted values for the blood flow field information at at least one position in the second vessel segment. The blood flow convergence relationship may include a conservation or equality relationship composed of blood inflow and outflow, such as the conservation of blood flow between inflow and outflow at the branching point. For example, at different positions in the adjacent or nearly located to the branching point in different vessel segments connected to the branching point, the predicted pressure values should be approximately equal, continuous, or otherwise satisfy physical constraint conditions. Exemplarily, each prediction interval may include upper and lower bounds, and updating the predicted values may involve adjusting the predicted values or prediction intervals to satisfy corresponding conservation or equation relationships between connected vessel segments.

It should be understood that throughout this disclosure, the models described for training methods can be applicable to prediction methods or determination methods in other embodiments disclosed herein, and the prediction methods or determination methods in the embodiments disclosed herein can use the models trained according to the training methods disclosed herein, or may also use other models or algorithms understood by those skilled in the art. For brevity, these repetitive or similar features are not reiterated.

Furthermore, it should be understood that although the operations are depicted in the various figures as being performed in a specific order, this should not be construed as requiring these operations to be performed in the specific order shown, or requiring all of the operations shown to be performed to achieve the desired results. For example, the two steps described in sequence in this document can be performed in the reverse order, or can be performed concurrently. Additionally, one or more steps may be omitted in one or more embodiments of the present disclosure.

Moreover, it should be understood that the methods for predicting or determining data involved in one or more embodiments disclosed herein are not methods for directly determining diagnostic results by doctors, but involve data processing or information processing in medical processes, the results of which can be used for reference by doctors to assist in their medical operations. It should be understood that the information processing methods, data prediction methods, determination methods, decision methods, etc., involved in one or more embodiments disclosed herein are executed by computers or devices containing computers.

It should be understood that throughout this disclosure, images, image data, or image sequences can be or include two-dimensional image data, or can be or include three-dimensional image data. Images, image data, or image sequences can be images directly acquired and stored or otherwise transmitted to terminal devices for user access. Images, image data, or image sequences can also be processed image data after various image processing. Images, image data, or image sequences may undergo other analysis processes (such as lesion feature or lesion presence analysis processes) and contain analysis results (such as delineation of regions of interest, segmentation results of tissues, etc.). It should be understood that the present disclosure is not limited thereto.

Figure 3B5 is a schematic diagram illustrating the blood flow field information determination device 360 according to an exemplary embodiment. The blood flow field information determination device 360 may include: an image acquisition unit 361, a lesion information acquisition unit 362, and a flow field information determination unit 363. The image acquisition unit 361 may be used to obtain vessel segment image data for the target vessel segment; the lesion information acquisition unit 362 may be used to obtain lesion information associated with at least one lesion area related to the target vessel segment. The flow field information determination unit 363 may be used to determine at least one blood flow field information associated with the target vessel segment based on the vessel segment image data and the lesion information.

When understood, the various modules of the device 360 shown in Figure 3B5 can correspond to the various steps in the method 340 described with reference to Figure 3B2. Therefore, the operations, features, and advantages described above with respect to method 340 and its variant examples apply equally to device 360 and its included modules. For brevity, certain operations, features, and advantages are not reiterated here.

According to one embodiment of the present disclosure, a computing device is provided, comprising a memory, a processor, and computer programs stored on the memory. The processor is configured to execute the computer programs to implement the steps of any one of the method embodiments described above.

According to one aspect of the present disclosure, a non-transitory computer-readable storage medium is provided, storing computer programs that, when executed by a processor, implement the steps of any one of the method embodiments described above.

According to one aspect of the present disclosure, a computer program product is provided, comprising computer programs that, when executed by a processor, implement the steps of any one of the method embodiments described above.

While specific modules have been discussed above with specific functions, it should be noted that the functions of the various modules discussed in this document can be divided into multiple modules, and/or at least some functions of multiple modules can be combined into a single module. The action of performing specific actions described for specific modules may include the specific module itself performing the action, or alternatively, another component or module may execute the action (or execute the action together with the specific module in a replacement or other manner). Therefore, the specific module performing the action may include the specific module itself performing the action and/or another module or component that the specific module calls or otherwise accesses to perform the action. For example, various modules or units described according to one or more embodiments disclosed herein may be combined into a single module or unit in some embodiments. Additionally, in some embodiments described in one or more embodiments of the present disclosure, two or more modules or units may be described in parallel, while in other embodiments, these modules and units may have one or more containment relationships. As used herein, the phrases "entity A initiates action B" or "entity A causes action B to be performed" may refer to entity A issuing instructions to perform action B, but entity A itself does not necessarily perform action B.

Figure 3D illustrates an exemplary vascular segment with a curved morphology, including exemplary lesions (e.g., plaques). The exemplary straightened image obtained after straightening processing is shown in Figure 3E. The flow rates Q1, Q2, and Q3 of individual vascular segments in the curved morphology vascular model or blood flow model shown in Figure 3D can respectively correspond to the flow rates Q1', Q2', and Q3' of individual vascular segments in the straightened morphology vascular model or blood flow model shown in Figure 3E. Before and after straightening processing, other unillustrated physical quantities such as viscosity, pressure, flow velocity, etc., can also correspond to each other, for example, being equal or satisfying specific transformation relationships. In the specific example shown in Figure 3E, where the straightened vascular segment model is depicted as a three-dimensional model, it could be represented by multiple two-dimensional images or image sequences. For instance, such multiple two-dimensional images or image sequences can be input into a network to generate corresponding flow field information. However, it is understood that the present disclosure is not limited to this.

According to some embodiments, obtaining straightened vascular segment models based on the vascular segment image data may include: obtaining the vascular centerline associated with at least one vascular branch based on the vascular segment image data; and modeling based on the vascular centerline to obtain the straightened vascular segment model. For example, the vascular centerline of the curved vessel can first be obtained, unfolded along the length dimension, and deformed into a straight line shape (e.g., cylinder, approximate cylinder, truncated cone, or cone), thereby obtaining the straightened vascular segment model. According to some embodiments, modeling based on the vascular centerline to obtain the straightened vascular segment model may include: obtaining vascular cross-sectional data corresponding to multiple points on the vascular centerline; and based on the vascular cross-sectional data and the vascular centerline, obtaining the straightened vascular segment model. For example, multiple points can be obtained using strategies such as fixed interval sampling, predetermined multiple sampling points, key points, bifurcation points, etc., and cross-sectional data corresponding to these points can be mapped to the unfolded centerline, thereby achieving vascular modeling in a straightened form. In other embodiments, transformation from curved vessel segment data to straightened vessel segment data (straightening processing) can be achieved through pre-trained models. It is understood that the above are only examples, and other vascular model transformation algorithms understandable to those skilled in the art or emerging in the future may also be employed for simplified calculations, and the present disclosure is not limited thereto.

Figure 3D2 depicts a flowchart illustrating a method 370 for determining human medical information according to exemplary embodiments. Method 370 can be performed at a client device (e.g., client device 110 shown in Figure 1), i.e., the entity executing the steps of method 370 can be client device 110 as shown in Figure 1. In some embodiments, method 370 can be performed at a server (e.g., server 120 shown in Figure 1). In some embodiments, method 370 can be executed by combining a client device (e.g., client device 110) and a server (e.g., server 120).

In the following text, each step of method 370 is described in detail.

Referring to Figure 3D2, at step 371, a predicted value of blood flow field information associated with the target vascular segment is obtained based on vascular segment image data about the target vascular segment.

At step 372, a predicted interval associated with the blood flow field information is obtained.

At step 373, at least one human medical information associated with the target vascular segment is determined based on the predicted interval.

Through the above method, human medical information can be obtained more accurately. For example, when calculating human medical information, referring to the predicted interval can reduce introduced errors and improve calculation accuracy.

According to some embodiments, the blood flow field can be a pressure field. For example, the determined at least one blood flow field information may include at least one of pressure, flow velocity, and flow rate. Thus, important physical quantities in the blood flow field can be obtained, and other physiological or pathological information related to the target vascular segment can be determined accordingly.

According to one or more embodiments of the present disclosure, the predicted interval associated with the blood flow field information can be provided in the form of an interval flow field, which can, for example, replace a fixed flow field and instead generate flow field information with interval ranges. For example, interval flow fields can be optimized based on branching, thickness, flow field parameters, etc. Additionally, interval flow fields can be obtained by predicting the most likely values and interval values. As another example, only interval values can be obtained, omitting the step of predicting the most likely values.

When diagnosing coronary artery disease, it is often necessary to assess the health of the coronary arteries. For example, functional assessment based on the fractional flow reserve (FFR) can be used. FFR refers to the ratio of the mean pressure in the narrowest part of a coronary artery under maximum myocardial perfusion to the mean pressure in the aorta at the beginning of the coronary artery during maximum myocardial perfusion. According to some embodiments, human medical information may include FFR information. For example, when the blood flow field is a pressure field or the determined blood flow field information includes pressure values at corresponding locations, FFR's predicted value can be obtained based on the blood flow field information. In other examples, other human medical information understandable to those skilled in the art related to the target vascular segment, such as plaque risk assessment values or surgical guidance, can be determined based on the blood flow field information, which may include blood pressure, flow velocity, shear stress, etc., and the present disclosure is not limited thereto.

As a specific non-limiting example, in the case where the required human medical information includes FFR values, in addition to pressure P information, predictions can be made for flow velocity v, flow rate Q, and/or viscosity η, and the predicted values of these physical quantities can be made to satisfy physical constraints, such as one or more fluid mechanics equations or dynamic equations. Such prediction models can predict more comprehensive physical quantities, thereby achieving a deeper understanding of physical relationships. It is understood that the present disclosure is not limited thereto. In other words, the method may include obtaining vascular segment images of the target vascular segment and obtaining predicted values of a first physical quantity and at least one second physical quantity associated with the blood flow field of the target vascular segment based on the vascular segment image data, where the predicted value of the first physical quantity and the predicted value of at least one second physical quantity satisfy at least one constraint condition. Human medical information regarding the target vascular segment can be determined based on the predicted value of the first physical quantity. For example, predicted values of the second physical quantity may not be used to determine human medical information but may be used solely to supervise the first physical quantity and/or the model itself based on physical constraint conditions. With such embodiments, the model output can conform to physical formulas, ensuring the accuracy and rationality of the model output at the physical level.

According to some embodiments, determining at least one human medical information associated with the target vascular segment based on the predicted interval can include: obtaining updated predicted values of the blood flow field information based on the predicted interval of the
blood flow field information; and determining at least one human medical information associated with the target vascular segment based on the updated predicted values.

In such embodiments, the predicted values of the blood flow field information (e.g., a part of the blood flow field information) can be updated based on the predicted interval, and human medical information can be determined based on the updated predicted values, thereby increasing the prediction accuracy.

According to some embodiments, the target vascular segment may include a first vascular segment and a second vascular segment, where the first vascular segment can be connected to the second vascular segment via a branching point. In such embodiments, determining the updated predicted values of the blood flow field information associated with the second vascular segment based on the predicted interval can include: determining the updated predicted values of the blood flow field information associated with at least one position in the second vascular segment based on the predicted interval of the blood flow field information associated with at least one position in the first vascular segment. For example, the first and second vascular segments can include one being a collateral vessel and the other being a main vessel. Alternatively, both the first and second vascular segments can be collateral vessels. In such embodiments, predictions for associated vessels can be updated based on vascular topology. Additionally, in such embodiments, it can be understood that obtaining the predicted values associated with the predicted interval may include determining predicted values and/or predicted intervals of the blood flow field information at one or more positions (e.g., one or more predicted points) in the first vascular segment, which can be sampled or selected based on predetermined or machine-automated resolutions, or additionally selected based on lesion locations, lesion positions, key point positions, etc., and the present disclosure is not limited thereto.

According to some embodiments, the first vascular segment and the second vascular segment can also be connected to a third vascular segment via the branching point, where the updated predicted values of the blood flow field information associated with at least one position in the second vascular segment can be based on the predicted interval of the blood flow field information associated with at least one position in the third vascular segment. One of the first, second, and third vascular segments can be a main vessel, and the others can be collateral vessels. The main vessel in the first, second, and third vascular segments can branch into two collateral vessels, or it can be formed by the convergence of two collateral vessels. For example, as shown in Figure 3D3A, vascular segments 3711 and 3712 merge into vascular segment 3713. Alternatively, the first, second, and third vascular segments can all be main vessels, and one of them can be a collateral vessel, such as shown in Figure 3D3B, where vascular segments 3721 and 3722 belong to the same main vessel and extend to collateral vessel 3723 at the branching point. It is understood that the above are only examples, and the present disclosure is not limited thereto.

For example, determining the updated predicted values of the blood flow field information associated with at least one position in the second vascular segment based on the predicted interval of the blood flow field information associated with at least one position in the first vascular segment can include: based on blood flow convergence relationships at the branching point, and based on the predicted interval of the blood flow field information at at least one position in the first vascular segment and the predicted interval of the blood flow field information at at least one position in the third vascular segment, determining the updated predicted values of the blood flow field information at at least one position in the second vascular segment.

Blood flow convergence relationships can include conservation or equality relationships formed by blood flow in and out, such as the conservation of blood flow between inflow and outflow branching points. For example, referring back to Figure 3D3A, in the direction of blood flow depicted, the blood flow between vascular segments 3711 and 3713 can satisfy Q1+Q2=Q3. Similarly, in different vascular segments connected to branching points, adjacent or overlapping positions near or at the branching point may have predicted pressure values, viscosity values, etc., that are approximately equal to each other, continuous, or otherwise satisfy physical constraint conditions. For example, each predicted interval can include upper and lower bounds, and updating the predicted values can include adjusting the predicted values or predicted intervals to ensure that the upper and lower bounds between the interconnected vascular segments satisfy the corresponding conservation or equation relationships.

Due to the complex structure of the human body and limitations in image acquisition conditions, vascular segment images often exhibit occlusion, distortion, overlap, etc., and may result in differences in image clarity, accuracy, and angiographic imaging degree relative to other vascular segments. Alternatively, certain vessels may be more twisted, fluctuate significantly with respiration and heartbeat, be smaller in size, or have blurred boundaries, leading to potential errors in the model's predictive capabilities. The disclosed method is particularly applicable to such situations. For example, predicted values and predicted intervals of superior vascular segments with clearer imaging and more stable morphologies can be used to update predicted values of inferior vascular segments with less clear imaging, occlusions, significant temporal fluctuations, etc., leveraging the connection between them.

Various types of vascular segment images can be used. For example, vascular segment images can be CT images. For example, vascular segment image data about the target vascular segment may be images acquired through digital subtraction angiography (DSA) and the present disclosure is not limited thereto. For example, vascular segment image data about the target vascular segment may be coronary CTA images acquired through computed tomography angiography (CTA). As another non-limiting example, the vascular segment image data can also be segmentation data for the target vascular segment, such as segmentation mask data. In such examples, any method understandable to those skilled in the art can be used to achieve image segmentation, and the present disclosure is not limited thereto.

According to some embodiments, obtaining the predicted interval associated with the blood flow field information may include: obtaining a fluctuation interval of at least one additional blood flow field information associated with the blood flow field information; and based on the fluctuation interval of the additional blood flow field information, obtaining the predicted interval associated with the blood flow field information through a fluid dynamics model regarding the target vascular segment.

According to some embodiments, the blood flow field information may include first blood flow field information and second blood flow field information, and obtaining the predicted interval associated with the blood flow field information may include: obtaining interfered predicted values of the first blood flow field information based on predicted values of the first blood flow field information; obtaining, based on the interfered predicted values of the first blood flow field information, the predicted interval associated with the second blood flow field information through a fluid dynamics model regarding the target vascular segment.

In one or more embodiments, the predicted interval can be calculated by applying fluctuations or interferences, but it can be understood that the present disclosure is not limited thereto. In other embodiments, the predicted interval or upper and lower bounds of the predictions can be output directly by the network or model used for generating predicted values, or other networks or models different from the prediction model described above can be used.

According to one or more embodiments of the present disclosure, in addition to or instead of outputting predicted flow field values, a range flow field can be output, wherein each flow field information is represented as a flow field range or cloud, thereby providing richer information input for subsequent calculations of human medical information.

In some illustrative non-limiting embodiments, vascular segment image data can be straightened image data, where the straightening process is used to straighten one or more curved vascular branches of the target vascular segment into non-curved vascular branch models. For example, straightened image data can be straightened images (or sequences of straightened images) where one or more curved vascular branches of the target vascular segment are straightened. The straightened image data can be or can be represented as a three-dimensional model. Since the flow field information of interest in medical image modeling is often known flow field information for specific points, deforming curved vessels into straight vessels in this case does not introduce measurable errors into the computation results. However, it can greatly simplify the complexity of vessel models, thereby simplifying modeling and computation processing, and increasing computational efficiency.

According to some embodiments, obtaining the straightened vascular segment model based on the vascular segment image data can include: obtaining the vascular centerline of at least one vascular branch based on the vascular segment image data; and modeling based on the vascular centerline to obtain the straightened vascular segment model. For example, the vascular centerline of curved vessels can be obtained first, then unfolded and deformed into a straight-line form (such as a cylinder, approximate cylinder, frustum, or cone), thereby obtaining the straightened vascular segment model. According to some embodiments, modeling based on the vascular centerline to obtain the straightened vascular segment model can include: obtaining vascular cross-sectional data corresponding to multiple points on the vascular centerline; and based on the vascular cross-sectional data and the vascular centerline, obtaining the straightened vascular segment model. For example, multiple points can be obtained based on a point selection strategy such as fixed intervals, predetermined multiple sampling points, key points, branching points, etc., and cross-sectional data corresponding to these points can be obtained. The corresponding cross-sectional data can be mapped to the unfolded centerline to achieve the modeling of straightened vessels. According to other embodiments, a pre-trained model can be used to transform curved vessel segment data into straightened vessel segment data (straightening process). It can be understood that the above are only examples, and other vessel model transformation algorithms that those skilled in the art can understand or may emerge in the future can also be used for simplified computation, and the present disclosure is not limited thereto.

According to some embodiments, obtaining predicted values of a first physical quantity and a second physical quantity in the blood flow field corresponding to the target vascular segment can include obtaining predicted values of the first physical quantity and the second physical quantity in the blood flow field corresponding to the target vascular segment based on lesion information related to at least one lesion area of the target vascular segment. According to some embodiments, the lesion information can be segmentation data of at least one lesion area, such as segmentation mask data.

As a specific non-limiting example, lesion information (e.g., lesion location) can be used as prompt input to the model for determining blood flow field information. As another non-limiting example, the model can be trained to additionally have the ability to predict lesion information, thereby enhancing the understanding of the current image.

Illustratively, using lesion information as a reference may include personalizing various thresholds related to the subject, imaging environment, contrast agent, imaging level, etc., with the segmentation of the lesion. For example, CT values (e.g., pixel color depth) between lesion and non-lesion areas can be used to calibrate the overall image tone level. As another example, using lesion information as a reference may include higher accuracy, higher precision, more computational power, and lower error threshold requirements for flow field prediction in lesion areas. As an additional or alternative example, using lesion information as a reference may include determining sampling points, prediction points, key points, etc., based on the lesion location. For example, the density of points for generating flow field information at various points can be determined based on the lesion size, matching the scale of the generated result with the lesion size. For example, key points can be determined based on the type and location of the lesion, where specific flow field information at these key points is more important for subsequent diagnosis and treatment. Continuing the example of FFR prediction from above, with the knowledge of the lesion location, the model can predict pressure field information with higher accuracy and specificity for upstream and downstream areas of the lesion (e.g., arterial stenosis), thereby obtaining more accurate estimates without wasting computational resources.

It can be understood that any method understandable to those skilled in the art can be used to train the models or networks required in one or more embodiments of the present disclosure, such as models or networks for predicting blood flow field information. For ease of understanding, a non-limiting example of a training method 380 is described in conjunction with Figure 3D4.

At step 381, vascular segment image data about the sample vascular segment is obtained.

At step 382, predicted values of the blood flow field information are obtained based on the vascular segment image data using a blood flow field prediction model.

At step 383, at least one first strategy is used to adjust the parameters of the blood flow field prediction model, wherein the first strategy is used to reduce the difference between the predicted values of the blood flow field information and the corresponding true values.

In some optional embodiments, step 382 may also include obtaining predicted intervals associated with the blood flow field information based on the vascular segment image data using a blood flow field prediction model, and step 383 may include a second strategy for supervising the predicted intervals. In other embodiments, the model for generating predicted intervals can be based on fluid dynamics formulas or trained separately.

Illustratively, the blood flow field information can be used to determine at least one human medical information associated with the sample vascular segment.

Additionally, parameters of the blood flow field prediction model can be adjusted based on additional strategies to satisfy at least one constraint condition between various physical quantities of the blood flow field information. Illustratively, the at least one constraint condition can be a physical constraint condition. For example, physical quantities satisfying constraint conditions may satisfy one or more physical equations or constraints represented in other forms at the physical level. As a specific non-limiting example, the true values of pressure P and velocity v can be used to estimate the true value of flow rate Q through physical equations, and the corresponding predicted values of
pressure, velocity, and flow rate can be compared with the corresponding true values. If they match, it can be considered that the physical constraint condition is satisfied.

It can be understood that throughout the present disclosure, the models described in the training method can be applicable to prediction methods or determination methods of other embodiments in the present disclosure, and the prediction methods or determination methods in the embodiments of the present disclosure can use models trained according to the training method in the embodiments of the present disclosure, or other models or algorithms understandable to those skilled in the art, and for brevity, these repetitive or similar features are not reiterated.

Furthermore, it can be understood that although each operation is depicted in each figure as being performed in a specific order, this should not be construed as requiring these operations to be performed in the specific order shown, nor should it be construed as requiring all operations shown to be performed to obtain the desired result. For example, the two steps described in order in this document can be performed in the reverse order, or they can be performed concurrently. Similarly, one or more steps in each embodiment of the present disclosure may be omitted.

Additionally, it can be understood that the methods for predicting or determining data described in one or more embodiments of the present disclosure are not methods for directly determining diagnostic results by doctors, but involve data processing or information processing processes in the medical process, the results of which can be used for reference by doctors to assist in their medical operations. It can be understood that the information processing methods, data prediction methods, determination methods, decision methods, etc., involved in one or more embodiments of the present disclosure are executed by computers or devices containing computers.

It can be understood that throughout the present disclosure, images, image data, or image sequences can be two-dimensional image data or three-dimensional image data. Images, image data, or image sequences can be directly acquired and stored or otherwise transmitted to terminal devices for user use. Images, image data, or image sequences can also be processed image data after various image processing. Images, image data, or image sequences may undergo other analysis processes (e.g., lesion feature analysis or lesion detection) and may contain analysis results (e.g., encirclement of regions of interest, segmentation results of tissues, etc.). It can be understood that the present disclosure is not limited thereto.

Figure 3D5 is a schematic diagram illustrating a human medical information determination device 390 according to an exemplary embodiment. The human medical information determination device 390 may include: a predicted value acquisition unit 391, a predicted interval acquisition unit 392, and an information determination unit 393. The predicted value acquisition unit 391 can be used to obtain predicted values of blood flow field information associated with the target vascular segment based on vascular segment image data about the target vascular segment. The predicted interval acquisition unit 392 can be used to obtain predicted intervals associated with the blood flow field information. And the information determination unit 393 can be used to determine at least one human medical information associated with the target vascular segment based on the predicted intervals.

It should be understood that the various modules of the device 390 shown in Figure 3D5 may correspond to the various steps described in the method 370 described with reference to Figure 3D2. Thus, the operations, features, and advantages described above with respect to the method 370 and its variant examples are also applicable to the device 390 and its included modules. For brevity, certain operations, features, and advantages are not reiterated here.

According to one aspect of the present disclosure, a computing device is provided, comprising a memory, a processor, and a computer program stored in the memory. The processor is configured to execute the computer program to implement at least one of the steps of the method embodiments described above.

According to one aspect of the present disclosure, a non-transitory computer-readable storage medium is provided, storing a computer program that, when executed by a processor, implements at least one of the steps of the method embodiments described above.

According to one aspect of the present disclosure, a computer program product is provided, comprising a computer program that, when executed by a processor, implements at least one of the steps of the method embodiments described above.

While specific modules have been discussed above with respect to specific functionalities, it should be noted that the functionalities of the various modules discussed herein can be divided into multiple modules, and/or some functionalities of multiple modules can be combined into a single module. The actions described for specific modules include the specific module itself performing the action or alternatively, the specific module calling or otherwise accessing another component or module to perform the action (or performing the action together with the specific module). Therefore, the specific module performing the action may include the specific module itself performing the action and/or another module accessed or called by the specific module performing the action. For example, various modules or units described in one or more embodiments of the present disclosure may be combined into a single module or unit in some embodiments. For example, in some embodiments, two or more modules or units described in one or more embodiments of the present disclosure may be described in parallel, while in other embodiments, these modules and units may have one or more containment relationships. As used herein, the phrases "entity A initiates action B" or "entity A causes action B to be performed" may refer to entity A issuing instructions to perform action B, but entity A itself does not necessarily perform action B.

It can be understood that any method understandable to those skilled in the art can be used to train the models or networks required in one or more embodiments disclosed herein. For ease of understanding, a non-limiting example of an illustrative training method 400 is described in conjunction with Figure 4.

At step 401, vascular segment image data about the sample vascular segment is obtained.

At step 402, predicted values of blood flow field information associated with the target vascular segment are obtained based on the vascular segment image data using a blood flow field prediction model.

At step 403, parameters of the blood flow field prediction model are adjusted based on at least a first strategy, wherein the first strategy is used to reduce the difference between the predicted values of the blood flow field information and the corresponding true values.

Illustratively, the blood flow field information can be used to determine at least one human medical information associated with the sample vascular segment.

Additionally, parameters of the blood flow field prediction model can be adjusted based on additional strategies to satisfy at least one constraint condition between various physical quantities. Illustratively, the at least one constraint condition can be a physical constraint condition. For example, physical quantities satisfying constraint conditions may satisfy one or more physical equations or constraints represented in other forms at the physical level. As a specific non-limiting example, the true values of pressure P and velocity v can be used to estimate the true value of flow rate Q through physical equations, and the corresponding predicted values of pressure, velocity, and flow rate can be compared with the corresponding true values. If they match, it can be considered that the physical constraint condition is satisfied.

According to one or more embodiments disclosed herein, a blood flow field information determination model or network is provided, which can generate corresponding blood flow field information based on original or processed image data about vascular segments, which can be used to determine human medical information. According to one or more embodiments disclosed herein, computational complexity and/or prediction accuracy can be reduced by simplifying images or using various reference information.

It can be understood that throughout the disclosure, the models described in the training method can be applicable to prediction methods or determination methods of other embodiments disclosed herein, and the prediction methods or determination methods in the embodiments disclosed herein can use models trained according to the training method in the embodiments disclosed herein, or other models or algorithms understandable to those skilled in the art, and for brevity, these repetitive or similar features are not reiterated.

Additionally, it can be understood that although each operation is depicted in each figure as being performed in a specific order, this should not be construed as requiring these operations to be performed in the specific order shown, nor should it be construed as requiring all operations shown to be performed to obtain the desired result. For example, the two steps described in order in this document can be performed in the reverse order, or they can be performed concurrently. Similarly, one or more steps in each embodiment disclosed herein may be omitted.

Furthermore, it can be understood that the methods for predicting or determining data described in one or more embodiments disclosed herein are not methods for directly determining diagnostic results by doctors, but involve data processing or information processing processes in the medical process, the results of which can be used for reference by doctors to assist in their medical operations. It can be understood that the information processing methods, data prediction methods, determination methods, decision methods, etc., involved in one or more embodiments disclosed herein are executed by computers or devices containing computers.

It can be understood that throughout the disclosure, images, image data, or image sequences can be two-dimensional image data or three-dimensional image data, and the disclosure is not limited thereto.

Figure 4A2 illustrates a flowchart of a blood flow field information determination method 410 according to an exemplary embodiment. Method 410 can be executed at a client device (e.g., client device 110 as shown in Figure 1), i.e., the entity performing the steps of method 410 can be client device 110 as shown in Figure 1. In some embodiments, method 410 can be executed at a server (e.g., server 120 as shown in Figure 1). In some embodiments, method 410 can be executed by a combination of a client device (e.g., client device 110) and a server (e.g., server 120).

In the following text, each step of method 410 is described in detail.

Referring to Figure 4A2, at step 411, vascular segment image data for the target vascular segment, which includes at least one vascular branch, is obtained.

At step 412, a straightened vascular segment model is obtained based on the vascular segment image data, wherein the straightened vascular segment model is used to represent at least one vascular branch in a non-curved form.

At step 413, at least one blood flow field information associated with the target vascular segment is determined based on the straightened vascular segment model.

Through the above method, the computation of blood flow field information can be simplified by using the straightened vascular segment model.

Due to the morphology of the human vasculature, one or more vascular branches may be curved in the vascular segment
image data. Modeling and computing based on such vascular segment images are often very complex. However, since the flow field information of interest in medical imaging is often known flow field information for specific points, straightening the curved vessels in this case does not introduce quantifiable errors into the calculation results, but greatly simplifies the modeling and calculation process.

Illustratively, the process of obtaining a straightened vascular segment model based on the vascular segment image data can be referred to as straightening processing. For example, straightening processing can be used to straighten one or more curved vascular branches of the target vascular segment into non-curved vascular branch models. For example, the image data after straightening processing can be referred to as straightened images, in which one or more curved vascular branches of the target vascular segment are straightened.

Figure 4A3A illustrates an exemplary vascular segment with a curved morphology, including an exemplary lesion (e.g., plaque). The exemplary straightened image obtained after straightening processing may appear as shown in Figure 4A3B. The flow rates Q1, Q2, and Q3 of each vascular segment in the curved vascular model or blood flow model shown in Figure 4A3A can correspond respectively to the flow rates Q1', Q2', and Q3' of each vascular segment in the non-curved vascular model or blood flow model shown in Figure 4A3B. Other physical quantities such as viscosity, pressure, velocity, etc., before and after straightening processing may correspond to each other, for example, being equal or satisfying specific transformation relationships. In the specific example shown in Figure 4A3B, the straightened vascular segment model is shown as a three-dimensional model, but it can be understood that the disclosure is not limited thereto.

According to some embodiments, obtaining a straightened vascular segment model based on the vascular segment image data can include obtaining the vascular centerline of at least one vascular branch based on the vascular segment image data, and modeling based on the vascular centerline to obtain the straightened vascular segment model. For example, the vascular centerline of curved vessels can be obtained first, expanded along the length dimension, and deformed into a straight line shape (e.g., cylinder, approximate cylinder, frustum, or cone), thus obtaining the straightened vascular segment model. According to some embodiments, obtaining the straightened vascular segment model based on the vascular centerline can include obtaining vascular cross-sectional data corresponding to multiple points on the vascular centerline, and obtaining the straightened vascular segment model based on the vascular cross-sectional data and the vascular centerline. For example, multiple points can be obtained using point selection strategies such as fixed interval sampling, predetermined multiple sampling points, keypoints, bifurcation points, etc., and the corresponding cross-sectional data can be mapped to the expanded centerline to achieve vascular modeling in a straightened form. According to other embodiments, transformation from curved vascular segment data to straightened vascular segment data (straightening processing) can be achieved using pre-trained models.

According to one or more embodiments disclosed herein, at least one straightened image can be generated based on original images. Illustratively, a portion of the image area containing vessels can be selected from the original images to generate straightened images. For example, areas containing vessels in the images can be selected while disregarding (e.g., cropping out, discarding) areas not containing vessels.

Illustratively, the method may further include obtaining at least one straightened vascular segment model based on the vascular segment image data, wherein each straightened vascular segment model corresponds to a branching point in the vascular segment image.

Illustratively, the method may further include, before obtaining at least one straightened vascular segment model based on the vascular segment image data: obtaining at least one sub-region of the vascular segment image, wherein each sub-region corresponds to each straightened vascular segment model. Illustratively, at least one sub-region of the vascular segment image is obtained based on a first selection strategy, wherein the first selection strategy is used to increase the proportion of the region corresponding to the side branch vessel in the target vascular segment in the corresponding sub-region. For example, a selection strategy that prioritizes regions containing branches can be used to increase the proportion of the branch region in the image space. For example, regions containing branches can be selected as much as possible, regions containing more branches can be selected, regions with a higher proportion of branch area relative to the total area can be selected, or a combination or trade-off of these options. Such illustrative strategies are based on the consideration that branches often have a significant impact on flow field predictions. Therefore, by prioritizing regions containing branches, more accurate FFR values can be calculated. For example, referring to Figure 4A3C, it may be more appropriate and accurate to model the region corresponding to box 4332 rather than box 4331. It can be understood that factors such as lesions, keypoints, analysis purposes, etc., can also be considered when selecting sub-regions, and the disclosure is not limited thereto.

Illustratively, the target vascular segment may include a first vascular segment, a second vascular segment, and a third vascular segment connected via a first branching point. For example, the flow rate of the first vascular segment may be the sum of the flow rates of the second and third vascular segments. For example, the first vascular segment may bifurcate into the second and third vascular segments, or the second and third vascular segments may converge into the first vascular segment. Alternatively, the first and second vascular segments may be two segments on either side of the branching point, and the third vascular segment may be a branching vessel that flows into or out of the first vascular segment. For example, as shown in Figure 4A3D, exemplary first vascular segment 4341, second vascular segment 4342, and third vascular segment 4343 are connected at branching point 4340.

Illustratively, obtaining a straightened vascular segment model based on the vascular segment image data may include obtaining a first straightened vascular segment model by considering the first vascular segment and the second vascular segment as the main vessels and the third vascular segment as a side branch vessel, with the first branching point as a reference point.

Illustratively, determining at least one blood flow field information associated with the target vascular segment based on the straightened vascular segment model may include obtaining at least one blood flow field information at at least one position point in the third vascular segment based on the first straightened vascular segment model.

Additionally or alternatively, blood flow field information at at least one position point in the first vascular segment and the second vascular segment can be obtained based on the first straightened vascular segment model.

In other words, in cases involving branching points, flow field information in the branch vessels can be calculated based on the straightened vascular model, and flow field information in the main vessel can also be calculated.

As a further embodiment, obtaining a straightened vascular segment model based on the vascular segment image data may include obtaining a second straightened vascular segment model by considering the first vascular segment and the third vascular segment as the main vessels and the second vascular segment as a side branch vessel, with the first branching point as a reference point. For example, blood flow field information at at least one position point in the third vascular segment based on the second straightened vascular segment model can also be obtained.

Referring back to Figure 4A3D, a specific non-limiting embodiment is described, wherein at least two straightened vascular models can be established for branching points associated with at
least three vascular segments, generating at least two sets of flow field prediction results, and determining the flow field prediction results of at least three vascular segments based on the two sets of flow field prediction results. For example, referring to Figure 4A3D, vascular segments 4341 and 4342 can be considered as main vessels, and a straightened image can be generated for them, and the corresponding flow field information for one or more position points in the branching vessel 4343, referred to as the first set of flow field information, can be calculated. Similarly, vascular segments 4341 and 4343 can be considered as main vessels, and a straightened image can be generated for them, and the corresponding flow field information for one or more position points in the branching vessel 4342, referred to as the second set of flow field information, can be calculated. The position points targeted by the first and second sets of flow field information may partially or completely overlap. At those overlapping position points, the final flow field information results can be calculated based on the first and second sets of flow field information, for example, by weighted averaging of the first and second sets of flow field information.

According to some embodiments, the method may further include obtaining lesion image data of at least one lesion area associated with the target vessel segment, wherein the straightened vessel segment model may also be based on the lesion image data. In some embodiments, the lesion information may be segmentation data of the at least one lesion area, such as segmentation mask data.

As a specific non-limiting example, lesion information (e.g., lesion location) can be input as prompt information into a model used to determine blood flow field information. As another non-limiting example, the model can be trained to additionally possess the ability to predict lesion information, thereby deepening the understanding of the current image. For example, using lesion information as a reference can involve personalizing various thresholds related to the current subject, imaging environment, contrast agent, and imaging level based on lesion segmentation. For instance, calibration of overall image color level can be performed using CT values (e.g., pixel color depth) of lesion and non-lesion areas. Similarly, the position of vessel branches and vessel cross-sectional width can be defined or adjusted based on the location of lesions. Additionally, using lesion information as a reference can involve higher accuracy, precision, computational power, and lower error threshold requirements for flow field prediction at lesion sites. As an additional or alternative example, using lesion information as a reference can involve determining sampling points, prediction points, key points, etc., based on lesion location. For instance, density of points for generating flow field information can be determined based on lesion size, ensuring that the scale of the generated result matches the lesion size. Moreover, key points can be defined based on lesion type, location, etc., where specific flow field information at these key points is more crucial for subsequent diagnosis and treatment. Continuing the example regarding FFR prediction from the previous context, in a scenario where lesion locations are obtained, the model can predict pressure field information of upstream and downstream regions of the lesion (e.g., arterial stenosis) area with higher accuracy, thus obtaining more accurate estimates without wasting computational resources.

According to some embodiments, the blood flow field can be a pressure field. Exemplary determinations of at least one blood flow field information may include at least one of pressure, flow velocity, and flow rate. Consequently, important physical quantities in the blood flow field can be obtained, which can be used to determine other physiological or pathological information related to the human body associated with the target vessel segment.

According to some embodiments, the method may further include obtaining at least one human medical information about the target vessel segment based on the at least one blood flow field information.

Diagnosing coronary heart disease often requires assessing the health of coronary arteries. For example, functional assessment can be based on the Fractional Flow Reserve (FFR). FFR refers to the ratio of the mean pressure distal to a coronary artery lesion (Pd) to the mean pressure proximal to the stenosis in the coronary artery (Pa) during maximal hyperemia. According to some embodiments, at least one human medical information may include information about the Fractional Flow Reserve (FFR). For instance, in cases where the blood flow field is a pressure field or the determined blood flow field information includes pressure values at corresponding locations, predicted values of FFR can be obtained based on the blood flow field information. In other examples, other human medical information about the target vessel segment that can be understood by those skilled in the art, such as plaque risk assessment, surgical guidance, etc., can be determined based on the blood flow field information, including blood pressure, flow velocity, shear stress, etc., and the disclosure is not limited thereto.

As a specific non-limiting example, in a scenario where the required human medical information includes the FFR value, predictions of variables such as velocity v, flow rate Q, and/or viscosity η can be made in addition to information about pressure P, and these predicted physical quantities can be constrained to satisfy physical constraints, such as one or more fluid dynamics equations or kinetic equations. Such predictive models can predict more comprehensive physical quantities, leading to a deeper understanding of physical relationships and therefore greater accuracy. In other words, the method may include obtaining vascular segment images of the target vessel segment and predicting values of a first physical quantity and at least one predicted value of a second physical quantity in the blood flow field corresponding to the target vessel segment based on the vascular segment image data, wherein the predicted value of the first physical quantity and the at least one predicted value of the second physical quantity satisfy at least one constraint. Human medical information about the target vessel segment can be determined based on the predicted value of the first physical quantity. Exemplary determination of the second physical quantity may not be used to determine human medical information but solely for supervising the first physical quantity and/or the model itself based on physical constraint conditions. In such embodiments, the output of the model can conform to physical formulas, ensuring the accuracy and rationality of the model output at the physical level.

Various types of vascular segment images can be used. Exemplary vascular segment images can be CT images. Exemplary vascular segment image data for the target vessel segment can be images acquired using digital subtraction angiography (DSA) methods, and the disclosure is not limited thereto. Exemplary vascular segment image data for the target vessel segment can be coronary CTA images acquired using computed tomography angiography. As another non-limiting example, the vascular segment image data for the target vessel segment can also be segmentation data, such as segmentation mask data. In such examples, any method that those skilled in the art can understand can be used to implement image segmentation, and the disclosure is not limited thereto.

According to some embodiments, the method may further include obtaining a prediction interval for the at least one blood flow field information. In such embodiments, human medical information about the target vessel segment may also be based on the prediction interval.

Exemplarily, the method may further include obtaining updated predicted values of the blood flow field information based on the prediction interval and at least one human medical information may be determined based on the updated predicted values. In such embodiments, the predicted values of blood flow field information (e.g., a part of the blood flow field information) can be updated based on the prediction interval, and human medical information can be determined based on the updated predicted values, thereby increasing the prediction accuracy.

Due to the complex structure of the human body and the limitations of image acquisition conditions, images of vascular segments often exhibit occlusion, distortion, overlap, etc., resulting in differences in image clarity, accuracy, and imaging quality relative to other vascular segments. Alternatively, models may have some degree of error in predicting vascular segments that are more twisted, exhibit significant morphological fluctuations with respiration and heartbeat, are smaller in size, or have blurred boundaries. For example, predicted values and prediction intervals for better-quality vascular segments, which are clearer in imaging and more stable in shape, can be used as a reference to update predicted values for poorer-quality vascular segments with issues such as unclear imaging, occlusion, significant temporal fluctuations, etc. For example, the target vessel segment may include a first vessel segment and a second vessel segment connected via a branching point. For instance, one of the first vessel segment and the second vessel segment can be a collateral vessel, while the other is a main vessel. Additionally, the first vessel segment and the second vessel segment may both be collateral vessels. In other words, updated predicted values of blood flow field information based on the prediction interval can include updated predicted values of blood flow field information at
at least one position in the second vessel segment based on the prediction interval of blood flow field information at at least one position in the first vessel segment.

Furthermore, it can be understood that obtaining a prediction interval associated with the predicted values can include determining predicted values and/or prediction intervals of the blood flow field information for one or more positions (e.g., one or more prediction points) in the first vessel segment separately. Such at least one position or prediction point, for example, can be sampled or selected based on predetermined or machine-automated resolution, or additionally selected based on lesion location, lesion position, key point position, etc., and the disclosure is not limited thereto.

Another example of updating associated vascular predictions based on vascular topology is when there are first, second, and third vessel segments in the target vessel segment connected via branching points. For example, one of the first, second, and third vessel segments can be a main vessel, while the others can be collateral vessels. The main vessels in the first, second, and third vessel segments can branch into two collateral vessels, or two collateral vessels can converge to form the main vessel. Alternatively, two of the first, second, and third vessel segments may both be main vessels, with the remaining one being a collateral vessel. In such examples, updated predicted values of blood flow field information at at least one position in the second vessel segment can be based on the prediction interval of blood flow field information at at least one position in the third vessel segment.

Exemplarily, determining updated predicted values of blood flow field information at at least one position in the second vessel segment based on the prediction interval of blood flow field information at at least one position in the first vessel segment can include: based on the blood flow convergence relationship at the branching point, and based on the prediction interval of blood flow field information at at least one position in the first vessel segment and the prediction interval of blood flow field information at at least one position in the third vessel segment, determining the updated predicted values of blood flow field information at at least one position in the second vessel segment. The blood flow convergence relationship can include conservation or equation relationships formed by the inflow and outflow of blood, for example, the blood flow volume at the inflow branching point should be conserved with the outflow branching point. Referring back to the exemplary illustration of Figure 4A3B, there could be Q1' = Q2' + Q3'. For example, at different positions near or almost located at branching points in different vessel segments connected to the branching point, the predicted pressure values should be roughly equal, continuous, or otherwise satisfy physical constraint conditions. Exemplarily, each prediction interval can include an upper limit and a lower limit, and updating the predicted values can include adjusting the predicted values or prediction intervals to satisfy corresponding conservation or equation relationships between interconnected vessel segments.

Exemplarily, determining at least one blood flow field information associated with the target vessel segment based on the straightened vessel segment model can be obtained through a pre-trained neural network. Any method that those skilled in the art can understand can be used to train the model or network required in one or more embodiments disclosed herein, such as a model or network for predicting blood flow field information. For ease of understanding, a non-limiting example training method 420 is described in conjunction with Figure 4A4.

At step 421, straightened vessel model data about a sample vessel segment is obtained.

At step 422, based on the straightened vessel model data, predicted values of at least one blood flow field information corresponding to the sample vessel segment are obtained through a blood flow field prediction model.

At step 423, parameters of the blood flow field prediction model are adjusted based on at least the first strategy, where the first strategy is used to reduce the difference between the predicted values of at least one blood flow field information and the corresponding true values.

Exemplarily, the at least one blood flow field information can be used to determine at least one human medical information about the sample vessel segment.

Exemplarily, additional strategies can be used to adjust the parameters of the blood flow field prediction model, where the additional strategies can be used to satisfy at least one constraint condition between various physical quantities of the at least one blood flow field information. Exemplarily, the at least one constraint condition can be a physical constraint condition. For example, physical quantities that satisfy constraint conditions can satisfy one or more physical formulas or physical constraint conditions expressed in other forms. As a specific non-limiting example, true values of flow rate Q can be estimated from true values of pressure P and velocity v through physical equations, and the corresponding predicted values of pressure, velocity, and flow rate can be compared with the corresponding true values, and if satisfied, it can be considered that the physical constraint condition is satisfied. According to such embodiments, the effect of making the model output conform to physical formulas can be achieved, thereby ensuring the accuracy and rationality of the model output at the physical level.

It can be understood that throughout the disclosure, the model described in the training method can be applied to prediction methods or determination methods of other embodiments disclosed herein, and the prediction method or determination method of embodiments disclosed herein can use the model trained according to the training method disclosed herein, or other models or algorithms that those skilled in the art can understand, and for brevity, these repetitive or similar features are not reiterated.

Furthermore, it can be understood that although various operations are depicted in various figures as being performed in a specific order, this should not be construed as requiring these operations to be performed in the specific order shown, nor should it be construed as requiring all operations shown to be performed to achieve the desired results. For example, two steps described in order in this document can be performed in reverse order, or concurrently. For example, one or more steps may be omitted in one or more embodiments disclosed herein.

Additionally, it can be understood that the methods for predicting or determining data involved in one or more embodiments disclosed herein are not methods directly determining diagnostic results by doctors, but involve data processing or information processing processes in medical processes, and their data processing results can be used for reference by doctors to assist in medical operations by doctors. It can be understood that the information processing method, data prediction method, determination method, decision method, etc., involved in one or more embodiments disclosed herein are executed by a computer or a device including a computer.

It can be understood that throughout the disclosure, images, image data, or image sequences can be or can include two-dimensional image data, or can be or include three-dimensional image data. Images, image data, or image sequences can be images directly acquired and stored or otherwise transmitted to terminal devices for user use. Images, image data, or image sequences can also be processed image data after various image processing. Images, image data, or image sequences can undergo other analysis processes (e.g., lesion feature or lesion existence analysis processes) and contain analysis results (e.g., circled regions of interest, segmentation results of tissues, etc.). It can be understood that the disclosure is not limited thereto.

Figure 4A5 illustrates a schematic diagram of a blood flow field information determination device 430 according to exemplary embodiments. The blood flow field information determination device 430 may include: an image acquisition unit 431, a model acquisition unit 432, and an information determination unit 433. The image acquisition unit 431 may be used to obtain vessel segment image data for a target vessel segment, wherein the target vessel segment includes at least one vessel branch. The model acquisition unit 432 may be used to obtain a straightened vessel segment model based on the vessel segment image data, where the straightened vessel segment model is used to represent the at least one vessel branch in a non-curved form. The information determination unit 433 may be used to determine at least one blood flow field information associated with the target vessel segment based on the straightened vessel segment model.

It should be understood that the various modules discussed above with reference to specific functions can be divided into multiple modules, and/or at least some functions of multiple modules can be combined into a single module. The actions performed by the specific modules discussed in this document include the specific modules themselves performing the actions, or alternatively, the specific modules may call or otherwise access another component or module to perform the actions (or perform the actions in conjunction with the specific modules). Therefore, the specific module that performs the action can include the specific module itself that performs the action and/or another module accessed by the specific module or accessed in another way that performs the action. For example, various modules or units described in one or more embodiments disclosed herein may be combined into a single module or unit in some embodiments. For example, in some embodiments described in one or more embodiments disclosed herein, two or more modules or units may be described in parallel, while in other embodiments, these modules and units may have one or more containment relationships. As used herein, the phrases "entity A initiates action B" or "entity A causes
action B to be performed" may refer to entity A issuing instructions to perform action B, but entity A itself does not necessarily perform action B.

It should also be understood that various technologies can be described in the general context of software, hardware components, or program modules. The various modules described above with reference to Figure 4A5 can be implemented in hardware or in hardware combined with software and/or firmware. For example, these modules can be implemented as computer program code/instructions configured to execute in one or more processors and stored in computer-readable storage media. Alternatively, these modules can be implemented as hardware logic/circuits. For example, in some embodiments, one or more of the modules or units described according to one or more embodiments disclosed herein can be implemented together in a System on Chip (SoC). The SoC can include an integrated circuit chip (including processors such as a Central Processing Unit (CPU), microcontrollers, microprocessors, Digital Signal Processors (DSPs), etc.), memory, one or more communication interfaces, and/or one or more components in other circuits), and may optionally execute received program code and/or include embedded firmware to perform functions.

According to one aspect of the present disclosure, a computing device is provided, comprising memory, a processor, and computer programs stored in the memory. The processor is configured to execute the computer programs to implement steps of any one embodiment of the methods described above.

According to one aspect of the present disclosure, a non-transitory computer-readable storage medium is provided, storing computer programs that, when executed by a processor, implement steps of any one embodiment of the methods described above.

According to one aspect of the present disclosure, a computer program product is provided, comprising computer programs that, when executed by a processor, implement steps of any one embodiment of the methods described above.

In the following, illustrative examples of such computing devices, non-transitory computer-readable storage media, and computer program products are described in conjunction with Figure 6.

Figure 5 is a schematic diagram illustrating a blood flow field information determination device 500 according to exemplary embodiments. The blood flow field information determination device 500 may include: an image acquisition unit 510 and an information determination unit 520. The image acquisition unit 510 may be used to obtain vessel segment images about the target vessel segment. The information determination unit 520 may be used to determine at least one blood flow field information for the target vessel segment based on the vessel segment images using a pre-trained neural network.

It should be understood that the various modules of the device 500 shown in Figure 5 can correspond to the various steps described in method 200 depicted in Figure 2. Therefore, the operations, features, and advantages described above with reference to method 200 and its variations are equally applicable to device 500 and its included modules. For brevity, certain operations, features, and advantages are not reiterated here.

According to one aspect of the present disclosure, a computing device is provided, comprising memory, a processor, and computer programs stored in the memory. The processor is configured to execute the computer programs to implement steps of any one embodiment of the methods described above.

According to one aspect of the present disclosure, a non-transitory computer-readable storage medium is provided, storing computer programs that, when executed by a processor, implement steps of any one embodiment of the methods described above.

According to one aspect of the present disclosure, a computer program product is provided, comprising computer programs that, when executed by a processor, implement steps of any one embodiment of the methods described above.

In the following, illustrative examples of such computing devices, non-transitory computer-readable storage media, and computer program products are described in conjunction with Figure 6.

Figure 6 illustrates an example configuration of a computing device 600 that can be used to implement the methods described in this document. For example, the server 120 and/or client devices 110 shown in Figure 1 may include architectures similar to the computing device 600. The blood flow field information determination devices/devices described above can also be implemented entirely or at least partially by the computing device 600 or similar devices or systems.

The computing device 600 can be various types of devices, such as servers for service providers, devices associated with clients (e.g., client devices), System on Chip (SoC), and/or any other suitable computing devices or systems. Examples of computing devices 600 include but are not limited to desktop computers, server computers, laptops or netbooks, mobile devices (e.g., tablets, cellular or other wireless phones (e.g., smartphones), notebooks, mobile stations), wearable devices (e.g., glasses, watches), entertainment devices (e.g., entertainment consoles, set-top boxes coupled to display devices, gaming consoles), televisions or other display devices, car computers, and so forth. Thus, the scope of the computing device 600 can range from full-resource devices with abundant memory and processor resources (e.g., personal computers, gaming consoles) to low-resource devices with limited memory and/or processing resources (e.g., traditional set-top boxes, handheld gaming consoles).

The computing device 600 may include at least one processor 602 capable of communicating with each other, such as via a system bus 614 or other appropriate connections. Memory 604, (multiple) communication interfaces 606, display device 608, other input/output (I/O) devices 610, and one or more large-capacity storage devices 612 may also be included.

The processor 602 can be a single processing unit or multiple processing units, all of which may include one or more computing units or multiple cores. The processor 602 may be implemented as one or more microprocessors, microcomputers, microcontrollers, digital signal processors, central processing units, state machines, logic circuits, and/or any other devices capable of manipulating signals based on operational instructions. In addition to other capabilities, the processor 602 may be configured to fetch and execute computer-readable instructions stored in memory 604, large-capacity storage device 612, or other computer-readable media, such as program code for operating systems 616, program code for applications 618, program code for other programs 620, etc.

Memory 604 and large-capacity storage device 612 are examples of computer-readable storage media for storing instructions executed by processor 602 to implement various functions described above. For example, memory 604 may generally include volatile and non-volatile memory (e.g., RAM, ROM, etc.), while large-capacity storage device 612 may generally include hard disk drives, solid-state drives, removable media, including external and removable drives, memory cards, flash memory, floppy disks, optical disks (e.g., CDs, DVDs), storage arrays, network-attached storage, storage area networks, and so forth. Memory 604 and large-capacity storage device 612 may be collectively referred to as memory or computer-readable storage media herein and may be non-transitory media capable of storing computer-readable, processor-executable instructions as computer program code to be executed by processor 602 configured to implement operations and functionalities described in examples herein.

Multiple program modules may be stored on large-capacity storage device 612. These programs include, for example, computer program logic (e.g., computer program code or instructions) for implementing components/functions including methods 200 and/or methods 400 (including any suitable steps of methods 200, 400) and/or additional embodiments described herein.

While modules 616, 618, 620, and 622 are illustrated as being stored in memory 604 of computing device 600 in Figure 6, modules 616, 618, 620, and 622 or portions thereof may be implemented using any form of computer-readable media accessible by computing device 600. As used herein, "computer-readable media" includes at least two types of computer-readable media, namely computer storage media and communication media.

Computer storage media include volatile and non-volatile, removable and non-removable media implemented by any method or technology for storage of information such as computer-readable instructions, data structures, program modules, or other data. Computer storage media include, but are not limited to, RAM, ROM, EEPROM, flash memory, or other memory technology, CD-ROMs, digital versatile discs (DVDs), or other optical storage devices, magnetic disks, magnetic tapes, magnetic disk storage devices, or other magnetic storage devices, or any other medium that can be used to store information for access by a computer device.

In contrast, communication media may embody computer-readable instructions, data structures, program modules, or other data in a modulated data signal, such as a carrier or other transmission mechanism. The computer storage media defined herein does not include communication media.

The computing device 600 may also include one or more communication interfaces 606 for exchanging data with other devices, such as over a network, via direct connections, and so forth, as discussed above. Such communication interfaces may include one or more of the following: any type of network interface (e.g., network interface card (NIC)), wired or wireless (such as IEEE 802.11 wireless LAN (WLAN)) wireless interfaces, Wi-MAX interfaces, Ethernet interfaces, Universal Serial Bus (USB) interfaces, cellular network interfaces, BluetoothTM interfaces, Near Field Communication (NFC) interfaces, and so forth. Communication interfaces 606 may facilitate communication within various networks and protocols, including wired networks (such as LANs, cables, etc.) and wireless networks (such as WLANs, cellular, satellite, etc.), the internet, and so forth. Communication interfaces 606 may also provide communication with external storage devices (not shown) such as storage arrays, network-attached storage, storage area networks, and so forth.

In some examples, a display device 608, such as a monitor, may be included for displaying information and images to a user. Other I/O devices 610 may be devices that receive various inputs from users and provide various outputs to users, and may include touch input devices, gesture input devices, cameras, keyboards, remote controls, mice, printers, audio input/output devices, and so forth.

Although the disclosure has been described and illustrated in detail in the accompanying drawings and preceding description, such description and illustration are to be considered illustrative and exemplary, not restrictive; the disclosure is not limited to the disclosed embodiments. By studying the drawings, the disclosure, and the appended claims, those skilled in the art will be able to understand and implement variations of the disclosed embodiments when practicing the subject matter to be protected as described herein. In the claims, the term "comprising" does not exclude other components or steps not listed, and the terms "a" or "an" do not exclude multiple. The mere fact that certain measures are recorded in mutually different dependent claims does not mean that the combination of these measures cannot be used to benefit.

## Claims

1. A method for determining blood flow field information, comprising:
obtaining vascular segment images regarding a target vascular segment; and
determining at least one blood flow field information for the target vascular segment based on the vascular segment images, through a pre-trained neural network.

2. The method according to claim 1, further comprising determining at least one type of blood flow field information based on an analysis purpose, where the analysis purpose indicates the category of human medical information to be obtained based on the vascular segment images, and
wherein the operation of determining at least one blood flow field information for the target vascular segment is based on the at least one type of blood flow field information;
wherein, optionally, the determined at least one type of blood flow field information corresponds to the category of the human medical information.

3. The method according to claim 2, wherein the determined at least one type of blood flow field information includes first type blood flow field information and second type blood flow field information, where the first type blood flow field information is used to generate the human medical information of the category, and the second type blood flow field information can satisfy one or more physical constraints with the first type blood flow field information.

4. The method according to any one of claims 1 to 3, wherein the at least one blood flow field information for the target vascular segment is also determined based on lesion information of at least one lesion area related to the target vascular segment;
wherein, optionally, the lesion information includes segmentation results related to the at least one lesion area.

5. The method according to any one of claims 1 to 4, further comprising determining at least one human medical information regarding the target vascular segment based on the at least one blood flow field information;
wherein, optionally, the human medical information includes Fractional Flow Reserve (FFR) information.

6. The method according to claim 5, further comprising obtaining a prediction interval for the at least one blood flow field information for the target vascular segment, and wherein the at least one human medical information regarding the target vascular segment is also based on the prediction interval.

7. The method according to claim 3, optionally in combination with any one of claims 4 to 6, wherein the physical constraint relationship is,
obtaining prediction values of a first physical quantity and a second physical quantity in the blood flow field corresponding to the target vascular segment based on vascular segment image data, wherein the prediction values of the first physical quantity and the second physical quantity satisfy at least one physical constraint condition; and
determining at least one human medical information regarding the target vascular segment based on the prediction value of the first physical quantity;

8. The method according to claim 7, wherein the blood flow field is a pressure field; and/or
wherein the first physical quantity is pressure, and the second physical quantity includes at least one of flow speed and flow rate; and/or
wherein the vascular segment image data is segmentation data for the target vascular segment; and/or
wherein the vascular segment image data is image data processed by straightening, wherein the straightening process is used to straighten one or more curved vascular branches of the target vascular segment into non-curved vascular branch models; and/or
wherein the prediction values of the first physical quantity and the second physical quantity are obtained through a pre-trained neural network; and/or
wherein obtaining prediction values of the first physical quantity and the second physical quantity in the blood flow field corresponding to the target vascular segment includes obtaining prediction values of the first physical quantity and the second physical quantity in the blood flow field corresponding to the target vascular segment based on lesion information of at least one lesion area related to the target vascular segment;
wherein, optionally, the lesion information includes segmentation results of the at least one lesion area.

9. The method according to any one of claims 1 to 8, wherein the method further includes obtaining a prediction interval for the first physical quantity, and wherein the at least one human medical information regarding the target vascular segment is obtained based on the at least one predicted quantity and the prediction interval.

10. The method according to any one of claims 1 to 9, wherein the method for determining blood flow field information may be:
obtaining vascular segment image data for the target vascular segment;
obtaining lesion information for at least one lesion area associated with the target vascular segment; and
determining at least one blood flow field information associated with the target vascular segment based on the vascular segment image data and the lesion information;
wherein, optionally, the at least one blood flow field information associated with the target vascular segment is determined through a pre-trained neural network, wherein the lesion information is input into the network as hint information;
and/or
wherein, optionally, the at least one blood flow field information associated with the target vascular segment is determined through a pre-trained neural network, and wherein obtaining lesion information for at least one lesion area related to the target vascular segment includes obtaining the lesion information through the neural network.

11. The method according to claim 6, optionally in combination with any of the claims 7 to 10, wherein the method for the prediction interval is:
based on vascular segment image data regarding the target vascular segment, obtaining a prediction value of blood flow field information associated with the target vascular segment;
obtaining a prediction interval associated with the blood flow field information; and
determining at least one human medical information associated with the target vascular segment based on the prediction interval;
wherein, optionally, the human medical information includes Fractional Flow Reserve (FFR) information.

12. The method according to claim 11, wherein determining at least one human medical information associated with the target vascular segment based on the prediction interval includes:
obtaining an updated prediction value of the blood flow field information based on the prediction interval; and
determining at least one human medical information regarding the target vascular segment based on the updated prediction value.

13. The method according to claim 12, wherein the target vascular segment includes a first vascular segment and a second vascular segment, wherein the first vascular segment is connected to the second vascular segment via a branching point, and
wherein obtaining the updated prediction value of the blood flow field information based on the prediction interval includes: determining an updated prediction value of the blood flow field information at at least one location in the second vascular segment based on a prediction interval of the blood flow field information at at least one location in the first vascular segment;
wherein, optionally, the first vascular segment and the second vascular segment are also connected to a third vascular segment via the branching point, and wherein the updated prediction value of the blood flow field information at at least one location in the second vascular segment is also based on a prediction interval of the blood flow field information at at least one location in the third vascular segment;
wherein, further optionally, determining an updated prediction value of the blood flow field information at at least one location in the second vascular segment based on a prediction interval of the blood flow field information at at least one location in the first vascular segment includes:
based on the blood flow convergence relationship at the branching point, and based on the prediction interval of the blood flow field information at at least one location in the first vascular segment and the prediction interval of the blood flow field information at at least one location in the third vascular segment, determining the updated prediction value of the blood flow field information at at least one location in the second vascular segment.

14. The method according to any one of claims 11 to 13, wherein obtaining a prediction interval associated with the blood flow field information includes:
obtaining a fluctuation interval of at least one additional blood flow field information associated with the blood flow field information; and
based on the fluctuation interval of the additional blood flow field information, obtaining the prediction interval associated with the blood flow field information through a fluid dynamics model regarding the target vascular segment.

15. A device for determining blood flow field information, comprising:
an image acquisition unit for obtaining vascular segment images regarding a target vascular segment; and
an information determination unit for determining at least one blood flow field information for the target vascular segment based on the vascular segment images, through a pre-trained neural network.

16. A computing device comprising:
a memory, a processor, and a computer program stored on the memory,
wherein the processor is configured to execute the computer program to implement the steps of the method according to any one of claims 1 to 14.

17. A non-transitory computer-readable storage medium, storing a computer program, wherein, when executed by a processor, the computer program implements the steps of the method according to any one of claims 1 to 14.

18. A computer program product, comprising a computer program, wherein, when executed by a processor, the computer program implements the steps of the method according to any one of claims 1 to 14.
